# FASCICULE DE BREVET EUROPEEN

(11) **EP 3 322 421 B1**
(45) Date de publication et mention de la délivrance du brevet: **10.06.2020**
(21) Numéro de dépôt: 16750383.8
(22) Date de dépôt: 12.07.2016
(51) Int. Cl.: A61K 31/49, C07D 401/14, C07D 249/04, G01N 33/543

(54) **COMPOSES INHIBITEURS DE PSEUDOMONAS AERUGINOSA**
PSEUDOMONAS AERUGINOSA-HEMMENDE VERBINDUNGEN
PSEUDOMONAS AERUGINOSA INHIBITOR COMPOUNDS

(30) Priorité: 13.07.2015 FR 1556639
(43) Date de publication de la demande: 23.05.2018
(73) Titulaire: Centre National de la Recherche Scientifique (CNRS), 75016 Paris (FR); Commissariat à l'Énergie Atomique et aux Énergies Alternatives, 75015 Paris (FR)
(72) Inventeur: WONG, Yung-Sing, 38400 Saint Martin d'Hères (FR); PLE, Sophie, 38640 Claix (FR); ATTREE, Ina, 38000 Grenoble (FR); BARETTE, Caroline, 38360 Sassenage (FR)
(74) Mandataire: Lavoix
(86) Numéro de dépôt international: PCT/EP2016/066550
(87) Numéro de publication internationale: WO 2017/009344

(56) Documents cités:
- WO-A1-2008/115118
- WO-A2-2009/140215
- S. PLE ET AL: "Cochaperone Interactions in Export of the Type III Needle Component PscF of Pseudomonas aeruginosa", JOURNAL OF BACTERIOLOGY, vol. 192, no. 14, 15 juillet 2010 (2010-07-15), pages 3801-3808, XP055253675, US ISSN: 0021-9193, DOI: 10.1128/JB.00117-10
- THIERRY IZOR ET AL: "Biogenesis, Regulation, and Targeting of the Type III Secretion System", STRUCTURE, vol. 19, no. 5, 1 mai 2011 (2011-05-01), pages 603-612, XP028206998, ISSN: 0969-2126, DOI: 10.1016/J.STR.2011.03.015 [extrait le 2011-04-22]
- M. GI ET AL: "A Drug-Repositioning Screening Identifies Pentetic Acid as a Potential Therapeutic Agent for Suppressing the Elastase-Mediated Virulence of Pseudomonas aeruginosa", ANTIMICROBIAL AGENTS AND CHEMOTHERAPY, vol. 58, no. 12, 1 décembre 2014 (2014-12-01), pages 7205-7214, XP055253679, US ISSN: 0066-4804, DOI: 10.1128/AAC.03063-14
- INES MANCINI ET AL: "Synthesis and bioactivity of linear oligomers related to polymeric alkylpyridinium metabolites from the Mediterranean sponge Reniera sarai", ORGANIC & BIOMOLECULAR CHEMISTRY, vol. 2, no. 9, 1 mai 2004 (2004-05-01), pages 1368-1375, XP055253683, GB ISSN: 1477-0520, DOI: 10.1039/B400782D

## Description

La présente invention porte sur des composés identifiés par criblage, en particulier en test ELISA, démontrant une efficacité *in vitro* en tant qu'inhibiteur de la machinerie responsable de la virulence chez *Pseusomonas aeruginosa.* C'est l'assemblage de l'injectisome de la sécrétion de type III de ces bactéries à Gram négatif qui est ciblé par les molécules de l'invention, notamment l'interaction protéine-protéine de PscE et PscG. Les molécules selon l'invention peuvent être obtenues facilement en peu d'étapes et permettent un accès simple à une grande variété d'analogues. L'invention vise aussi une méthode d'identification de composés capable d'inhiber la machinerie de virulence de *Pseudomonas aeruginosa,* un dispositif permettant la mise en œuvre de cette méthode d'identification. Le champ d'application concerne le traitement préventif et curatif des pathologies à *Pseudomonas aeruginosa,* en particulier les pathologies nosocomiales et ouvre la porte vers une nouvelle antibiothérapie mettant à disposition des composés rendant les bactéries inoffensives sans les détruire, évitant de ce fait toute pression de sélection induisant l'apparition de forme de résistance.

*Pseudomonas aeruginosa* appartient à une famille de pathogènes bactériens appelés « ESCAPE » (*E. faecium, S. aureus, Clostridium difficile, A. baumannii, Pseudomonas aeruginosa, Enterobacteriaceae*)*,* ayant la capacité d'« échapper » aux traitements antibiotiques actuels (L. R. Peterson. Bad bugs, no drugs: no ESCAPE revisited. Clin. Infect. Dis. 2009, 49, 992-993). Il est omniprésent dans la nature et sa capacité à s'adapter à différents milieux en disposant d'une panoplie d'effecteur de sécrétion en fait un pathogène humain opportuniste efficace. L'infection due à *Pseudomonas aeruginosa* peut être soit chronique, soit aigüe, dépendant de la pathologie sous-jacente ou de l'état immunitaire de l'individu. Lié à l'accroissement de formes de résistance multiple aux antibiotiques classiques, le traitement des infections liées à *Pseudomonas aeruginosa,* notamment dans le milieu hospitalier, représente aujourd'hui un grave problème de santé publique (F. Barbier, M. Wolff. Multi-drug resistant Pseudomonas aeruginosa: towards a therapeutic dead end? Med. Sci. (Paris) 2010, 26, 960-968). Dans l'optique d'une nouvelle thérapie antibactérienne, de nouvelles cibles spécifiques aux bactéries sont à l'étude, portant notamment sur le mécanisme de virulence pour développer un traitement alternatif aux infections bactériennes (F. Barbier, M. Wolff. Multi-drug resistant Pseudomonas aeruginosa: towards a therapeutic dead end? Med. Sci. (Paris) 2010, 26, 960-968).

Lors de la phase d'infection aigüe, *Pseudomonas aeruginosa* utilise une nanomachine de sécrétion de type III (T3S) hautement conservée. Quatre cytotoxines, ExoS, ExoT, ExoY et ExoU sont ainsi injectées, *via* l'aiguille de sécrétion, dans le cytoplasme de la cellule eucaryote fragilisant ainsi l'hôte ce qui permet l'invasion et l'instauration de l'infection (. R. Hauser. The type III secretion system of Pseudomonas aeruginosa: infection by injection. Nat. Rev. Microbiol. 2009, 7, 654-665). La machinerie T3S est une cible de choix comme l'atteste des travaux actuellement en cours et qui ciblent un de ces composants, PcrV, avec une approche thérapeutique basée sur les anticorps. En particulier, des fragments d'anticorps de haute affinité dirigés contre PcrV sont en développement par la société pharmaceutique KaloBios pour le traitement des infections liées à *Pseudomonas aeruginosa.*

Pour leur virulence, et aux premiers stades de l'infection, ces bactéries synthétisent au niveau de leur membrane bactérienne la nanomachine de virulence de type T3S, ou encore appelée injectisome de sécrétion de type III, qui permet d'injecter dans des cellules cibles des toxines, molécules destinées à perturber le fonctionnement de la cellule infectée et visant *in fine* à la détruire.

La nanomachine de virulence T3S, ou machinerie de virulence T3S, ou encore désignée T3S, est composée de protéines fonctionnellement conservées exportant des composants d'assemblage macromoléculaire à travers les deux membranes bactériennes (G. R. Cornelis. The type III secretion injectisome. Nat. Rev. Microbiol. 2006, 4, 811-825). Dans *Pseudomonas aeruginosa,* comme chez d'autres bactéries, T3S peut être divisé en trois sous-ensembles : la base, l'aiguille et le translocon, chacun composé de plusieurs macromolécules interagissant entre elles. Le translocon est la partie la plus éloignée de l'appareil T3S. Il est composé de deux protéines hydrophobes PopB et PopD qui s'associent à et avec la membrane de la cellule hôte. Cet assemblage permet le passage des toxines à travers le plasma membranaire. Dans la proximité du translocon et en interaction avec l'aiguille de sécrétion, PcrV crée le lien entre les deux entités.

PscF est le principal constituant de l'aiguille sécrétrice de 80 nM de long. L'inactivation génétique de gènes codant pour PscE et PscG conduit à des souches non-cytotoxiques sur cellules eucaryotes, due à l'absence d'activité T3S (M. Quinaud, J. Chabert, E. Faudry, E. Neumann, D. Lemaire, A. Pastor, S. Elsen, A. Dessen, I. Attree. The PscE-PscF-PscG complex controls type III secretion needle biogenesis in Pseudomonas aeruginosa. J. Biol. Chem. 2005, 280, 36293-36300)· La résolution de structure cristalline du complexe PscE-PscF-PscG a contribué grandement à la compréhension de leurs fonctions (M. Quinaud, S. Ple, V. Job, C. Contreras-Martel, J. P. Simorre, I. Attree, A. Dessen. Structure of the heterotrimeric complex that regulates type III secretion needle formation. Proc. Natl. Acad. Sci. U. S. A. 2007, 104, 7803-7808). PscG est une protéine qui interagit directement avec la partie hydrophobe de l'hélice C-terminale de PscF, lui-même nécessaire à la polymérisation de la protéine en aiguille. Il est intéressant de noter que PscG interagit avec PscE par une interaction hydrophobe sur une surface de 1300 Å². Des études biochimiques *in vitro* réalisées en parallèle avec des expériences *in vivo* sur *Pseudomonas aeruginosa* ont montré que PscE est une co-chaperone de PscG, et que l'absence de PscE mène à la déstabilisation de l'interaction PscG-PscF (S. Ple, V. Job, A. Dessen, I. Attree. Cochaperone interactions in export of the type III needle component PscF of Pseudomonas aeruginosa. J. Bacteriol. 2010, 192, 3801-3808). Des mutations simples et doubles points introduites dans la zone d'interaction entre PscE et PscG ont abouti à une souche de *Pseudomonas aeruginosa* présentant un phénotype non-cytotoxique.

Sur l'ensemble des études réalisées sur les systèmes de sécrétion de type III, dont celles conduites avec d'autres bactéries pathogènes, il ressort que de petites molécules de synthèse, ainsi que des produits d'origine naturelle ont été capables d'inhiber le pouvoir de virulence en tests cellulaires (L. K. Tsou, P. D. Dossa, H. C. Hang. Small molecules aimed at type III secretion systems to inhibit bacterial virulence. Med. Chem. Commun. 2013, 4, 68-79). Les salicylidènes acylhydrazides ont été parmi les premiers à montrer une efficacité sur le système T3S (WO 2006/132583). Leur mode d'action reste à être élucidé, bien que les effets liés à leur propriété chélatante du fer, des effets inhibiteurs putatifs de l'expression de TS3 ou des effets entraînant une diminution de la taille des aiguilles sécrétrices ont été avancées. Les salicylanilides ont été décrits comme étant une autre famille de composés efficaces sur T3S (M. K. Dahlgren, A. M. Kauppi, I.-M. Olsson, A. Linusson, M. Elofsson. Design, Synthesis, and Multivariate Quantitative Structure-Activity Relationship of Salicylanilides-Potent Inhibitors of Type III Secretion in Yersinia. J. Med. Chem. 2007, 50, 6177-6188). D'autres chélatants ont été identifiés par criblage, tels que la pyridine. D'autres familles de composés ont été découverts plus récemment, telles que les phenoxyacetamides (WO 2010/118046 A1), les 7-méthyl amine quinolines (WO 2008/115118) et les thiazolidinones (WO 2009/137133). Par ailleurs, des produits naturels, souvent complexes, ont aussi montré des propriétés spécifiques sur l'inhibition de T3S (K. Kimura, M. Iwatsuki, T. Nagai, A. Matsumoto, Y. Takahashi, K. Shiomi, S. Omura, A. Abe. A small-molecule inhibitor of the bacterial type III secretion system protects against in vivo infection with Citrobacter rodentium. J. Antibiot. 2011, 64, 197-203).

La présence d'un T3S fonctionnel est souvent associée au décès chez des patients présentant des infections respiratoires et systémiques provoquées par *Pseudomonas aeruginosa.* T3S semble contribuer au développement de pneumonie grave en empêchant la capacité de l'hôte à contenir l'infection bactérienne au niveau des poumons. Ceci permet non seulement à *Pseudomonas aeruginosa* de persister dans le poumon, mais il facilite également une surinfection par d'autres espèces de bactéries. Bien que plusieurs agents antibactériens soient efficaces contre *Pseudomonas aeruginosa,* les forts taux de mortalité associés aux infections à *Pseudomonas aeruginosa,* même chez les patients hospitalisés recevant des antibiotiques reflètent le besoin de nouveaux agents thérapeutiques. Les antibiotiques bactériostatiques et bactéricides conventionnels semblent en outre insuffisants pour combattre ces infections, et les nouvelles approches de traitement telles que des inhibiteurs des machineries de virulence de *Pseudomonas aeruginosa* peuvent s'avérer utile en tant que thérapies adjunctives.

C'est dans ce contexte que les inventeurs ont eu l'idée de développer un test ELISA permettant de cribler des petites molécules capables d'inhiber les interactions entre les deux protéines chaperones PscE et PscG.

Pour cela, les protéines individuelles PscE et PscG ont été purifiées à partir d'*E*. *coli.* Un test d'ELISA a été développé en fixant une de deux protéines au niveau de la surface d'un support solide, telle qu'une boite de microtitration à 96 puits. Suite au lavage, la deuxième protéine est ajoutée et l'interaction est révélée par les anticorps spécifiques et une réaction colorimétrique, grâce à un anticorps secondaire couplé à un substrat chromogénique ou fluorescent. C'est ainsi la première fois qu'un criblage de type chemogénomique inverse est effectué sur le système de T3S, à savoir que des inhibiteurs puissent être identifiés d'abord au niveau du complexe de protéines (chaperone PscE-PscG) pour ensuite rechercher l'effet phénotypique sur la cellule (inhibition de l'injectisome sur cellule). Plusieurs chimiothèques, ont été criblées afin d'identifier les composés capables d'inhiber l'interaction PscE-PscG et donc d'inhiber la machinerie de virulence de *Pseudomonas aeruginosa* et *in fine* de pouvoir prévenir ou traiter une infection pathogène causée par *Pseudomonas aeruginosa.*

Ple et al J. Bacteriology vol.192, n°.14, 2010, 3801-3808 ; Izor et al Structure vol.19, n°.5, 2011, 603-612 et WO 2008/115118 décrivent des composés capables d'inhiber la sécrétion de type III.

Gi et al Antimicrobial agents and chemotherapy vol.58, n°.12, 2014, 7205-7214 ; WO 2009/140215 ; Mancini et al Organic and Biomolecular chemistry vol.2, n°.9, 2004, 1368-1375 décrivent des composés capables de traitement des infections à *P.aeruginosa.*

Il est décrit un procédé d'identification de molécules inhibitrice de la machinerie de virulence de *Pseudomonas aeruginosa,* un dispositif pour l'identification d'une molécule inhibitrice de la machinerie de virulence de *Pseudomonas aeruginosa,* des composés nouveaux inhibiteurs de la machinerie de virulence de *Pseudomonas aruginosa,* des composés pour leur utilisation pour prévenir et/ou traiter une infection pathogène causée par *Pseudomonas aeruginosa* ainsi que des compositions pharmaceutiques pour prévenir et/ou traiter une infection pathogène causée par *Pseudomonas aeruginosa.*

La présente invention concerne un procédé d'identification d'une molécule inhibitrice de la machinerie de virulence de Pseudomonas aeruginosa comprenant une étape d'évaluation de la capacité de ladite molécule à inhiber l'interaction PscE/PscG. Ainsi dans un premier mode de réalisation, la présente invention vise un composé de formule (A) :

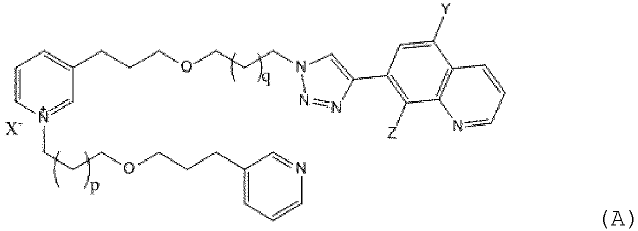

Dans lequel :
X est un halogène ;
Y est un halogène ;
Z est un groupement hydroxy, amine, ou un groupement -OR₁ dans lequel R₁ est un alkyle en C₁-C₄ ou un acyle en C₁-C₄ ;
   p est un nombre entier et 13 > p ≥ 2 ; et
q est un nombre entier et 13 > q ≥ 2, avec préférentiellement p+q <23 ;
à la condition que :
si p =4, alors q ≠ 8 ;
si p =10, alors q ≠ 4 ; et
si p =8, alors q ≠ 6.

Par « alkyle » ou « radical alkyle », on entend au sens de la présente invention une chaîne hydrocarbonée aliphatique linéaire ou ramifiée saturée et comprenant le nombre d'atomes de carbone spécifié. On peut par exemple citer, le méthyle, l'éthyle et le propyle.

Par « acyle » ou « radical acyle », abrégé en « Ac », on entend un radical où un atome de carbone est lié à un oxygène par une double liaison, à la structure de la molécule (A) par une liaison simple et à un radical alkyle, en l'espèce dans le cadre de la présente invention un radical alkyle C1-C4.

L'expression halogène définit un atome choisi dans le groupe constitué par le Chlore, le Fluor, le Brome et l'Iode.

De manière avantageuse, X est un Chlore.

De manière avantageuse Y est un Chlore.

D'autres sels pharmaceutiquement acceptables sont aussi possibles et X peut représenter un hydrogensulfate (HSO4-), un acide Trifluoroacetique (TFA-), ou un acide acétique par exemple.

De manière avantageuse R1 est un méthyl.

De manière avantageuse, dans un second mode de réalisation, le composé (A) selon le premier mode de réalisation est caractérisé par le fait que :
- Z est -OH ou OAc ;
- Y est Cl ;
- X est Cl ; et
- p et q sont des nombres pairs avec préférentiellement 8 ≥ p ≥ 2 et 10 ≥ q ≥ 2.

Dans un autre mode de réalisation de la présente invention, le composé (A) est caractérisé par le fait que :
- X est un Chlore, Y est un Chlore, R1 est un méthyl,
- p =2 et q =2, 4 ou 10, préférentiellement 4 ;
- p =4 et q =2, 4 ou 6, préférentiellement 4 ;
- p =6 et q =2, 4, 6 ou 10, préférentiellement 4 ; ou
- p =8 et q =4 ou 8, préférentiellement 4.

Dans un mode de réalisation avantageux, le composé (A) est caractérisé par le fait que X est un Chlore, Y est un Chlore, R1 est un méthyl, p=2 et q=4.

Dans un mode de réalisation avantageux, le composé (A) est caractérisé par le fait que X est un Chlore, Y est un Chlore, R1 est un méthyl, p=4 et q=4.

Dans un mode de réalisation avantageux, le composé (A) est caractérisé par le fait que X est un Chlore, Y est un Chlore, R1 est un méthyl, p=6 et q=4.

Dans un mode de réalisation avantageux, le composé (A) est caractérisé par le fait que X est un Chlore, Y est un Chlore, R1 est un méthyl, p=4 et q=6.

Dans un mode de réalisation avantageux, le composé (A) est caractérisé par le fait que X est un Chlore, Y est un Chlore, R1 est un méthyl, p=8 et q=4.

Dans un mode de réalisation avantageux, le composé (A) est caractérisé par le fait que X est un Chlore, Y est un Chlore, R1 est un méthyl, p=8 et q=8.

Dans un mode de réalisation avantageux, le composé (A) est caractérisé par le fait que X est un Chlore, Y est un Chlore, R1 est un méthyl , p=6 et q=10.

C'est aussi un objet de la présente invention que de fournir un procédé de fabrication d'un composé de formule (A) comprenant les étapes suivantes :
Fournir un composé de formule (B) : 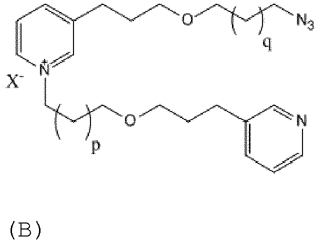
dans lequel X, p et q sont tels que définis plus avant,
   qui est mis en réaction en présence d'un catalyseur, préférentiellement du cuivre, avec le composé de formule (C): 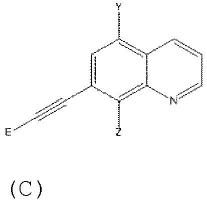
dans lequel Y et Z sont tels que définis ci avant,
et E est un groupement partant.

Par l'expression « groupement partant », on entend comprend un groupe qui peut être déplacé facilement par un nucléophile qui a une plus grande affinité pour l'atome de carbone chargé positivement auquel ledit groupe partant est fixé.

Dans un mode de réalisation particulier, le groupement partant est choisi dans le groupe constitué par le triméthylesilyle, le Tri-isopropyl silyle (TIPS) ou dimethyl alcohol par exemple.

C'est aussi un autre objet de la présente invention que de fournir un composé de formule (D) : 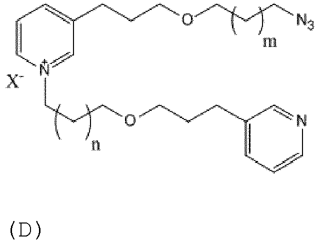 caractérisé en ce que :
X est un halogène ;
m est un nombre entier et 13 > m > 2 ; et
n est un nombre entier et 13 > n ≥ 2, préférentiellement 23 > m+n.

L'expression halogène définit un atome choisi dans le groupe constitué par le Chlore, le Fluor, le Brome et l'Iode.

De manière avantageuse, X est un Chlore.

D'autres sels pharmaceutiquement acceptables sont aussi possibles et X peut représenter un hydrogensulfate (HSO4-), un acide Trifluoroacetique (TFA-), ou un acide acétique par exemple.

Dans un mode de réalisation particulier, le composé de formule (D) selon l'invention caractérisé en ce que :
- X est Cl ;
- m et n sont des nombres pairs ; et/ou
- préférentiellement m+n > 6

C'est aussi un objet de la présente invention que de fournir un composé de formule (D) selon un mode de réalisation tel qu'illustré précédemment caractérisé en ce que :
- m =4 et n =6 ou 8, préférentiellement 6 ;
- m =6 et n =8 ou 10, préférentiellement 10 ;
- m =8 et n =4, 6, 8, ou 10, préférentiellement 6 ou 10,
- m =10 et n= 4 ou 10, préférentiellement 10.

Dans un mode de réalisation particulier, le composé (D) est caractérisé en ce que X est un chlore, m=4 et n= 2.

Dans un mode de réalisation particulier, le composé (D) est caractérisé en ce que X est un chlore, m=4 et n= 6.

Dans un mode de réalisation particulier, le composé (D) est caractérisé en ce que X est un chlore, m=6 et n= 4.

Dans un mode de réalisation particulier, le composé (D) est caractérisé en ce que X est un chlore, m=10 et n= 2.

Dans un mode de réalisation particulier, le composé (D) est caractérisé en ce que X est un chlore, m=4 et n= 8.

Dans un mode de réalisation particulier, le composé (D) est caractérisé en ce que X est un chlore, m=6 et n= 8.

Dans un mode de réalisation particulier, le composé (D) est caractérisé en ce que X est un chlore, m=8 et n= 8.

Dans un mode de réalisation particulier, le composé (D) est caractérisé en ce que X est un chlore, m=10 et n= 6.

Dans un mode de réalisation particulier, le composé (D) est caractérisé en ce que X est un chlore, m=10 et n= 8.

Dans un mode de réalisation particulier, le composé (D) est caractérisé en ce que X est un chlore, m=10 et n= 10.

C'est aussi un autre objet de la présente invention que de fournir des composés de formule (A) et/ou (D) en tant que médicament.

Plus particulièrement la présente invention concerne les composés de formule (A) et/ou (D) pour leur utilisation pour prévenir et/ou traiter une infection pathogène causée par *Pseudomonas aeruginosa.*

Dans un mode de réalisation particulièrement avantageux de réalisation de l'invention, l'infection pathogène traitée ou prévenue par le composés (A) et/ou (D) selon l'invention est une infection nosocomiale, préférentiellement choisie parmi les infections des yeux, des plaies, en particulier suite à une brûlure et/ou une opératoire, des urines, en particulier de la vessie ou de l'urètre par exemple après sondage, du système gastro-intestinale, d'un poumon en particulier après bronchoscopie, des méningites en particulier d'inoculation, des septicémies.

Ces composés selon l'invention ont démontré une efficacité en tant qu'inhibiteur de la machinerie responsable de la virulence chez *Pseudomonas aeruginosa.* Ces molécules ciblent l'assemblage de l'injectisome de la sécrétion de type III de ces bactéries et plus particulièrement l'interaction protéine-protéine PscE-PscG telle que démontré ici dans le test de criblage aussi objet de la présente invention. C'est la première fois que des composés de synthèse ont été identifiés comme inhibiteurs de ce mécanisme d'action empêchant la formation de l'injectisome. Ces molécules sont nouvelles et sont obtenues facilement en peu d'étapes (4 à 5 étapes), avec un accès simple à une grande variété d'analogues. Le champ d'application concerne une nouvelle antibiothérapie qui rendrait les bactéries inoffensives sans induire d'effet bactéricide, évitant de ce fait toute pression de sélection induisant l'apparition de forme de résistance.

La présente invention vise aussi une méthode permettant de cribler des molécules capables d'inhiber les interactions entre les deux chaperones PscE et PscG et par là même d'inhiber la machinerie responsable de la virulence chez *Pseudomonas aeruginosa.* Pour cela, tout d'abord les protéines individuelles PscE et PscG ont été purifiées à partir d'E. *coli.* Un test d'ELISA a été développé afin d'évaluer l'interaction protéine-protéine et l'inhibition de celle-ci par le composés testés. La réalisation de ce test débute en fixant une de deux protéines sur un support, tel que le matériau d'une boite à 96 puits. Suite au lavage, la deuxième protéine est ajoutée et l'interaction peut être révélée.

La mise en évidence de l'intéraction PscE-PscG ainsi que de l'inhibition de cette interaction peut être réalisée par divers moyens biochimiques à disposition d'un homme du métier pour la caractérisation des interactions protéine-protéine. Dans un mode particulier, l'interaction protéine-protéine est évaluée par des anticorps spécifiques et par une réaction colorimétrique, grâce à un anticorps secondaire couplé. Plusieurs chimiothèques et une vingtaine de produits naturels commerciaux peuvent être criblé manuellement ou de façon automatique afin de déterminer les composés possédant l'activité désirée. En plaçant la limite vers 50% d'inhibition à une concentration de 50 µM de la molécule testée, les résultats de ce premier criblage ont révélé 17 composés (Tableau 2) présentant une capacité d'inhibition permettant de conclure positivement quant à leur capacité à inhiber *Pseudomonas aeruginosa.* Cette valeur de % d'inhibition peut être bien sûr placée à un niveau inférieur et ne sert ici que de valeur guide informative.

Ainsi un composé testé pourra être retenu comme inhibiteur de la machinerie de virulence de *Pseudomonas aeruginosa* à partir d'un pourcentage d'inhibition de l'interaction PscE/PscG d'au moins 20%, au moins 30%, au moins 40%, au moins 50%, particulièrement au moins 60%, plus particulièrement au moins 70%, et plus particulièrement encore au moins 80%, voire plus particulièrement au moins 90% ou au moins 95%.

Les composés identifiés par cette méthode sont l'acide pentétique, le clioquinol, le myricétine, le bensérazide, le gossypol, un sel de 3-alkylpyridium 6 (3-APS), et l'héderagénine.

Dans la perspective de développer des composés structuralement originaux, différents de ceux déjà décrits, les efforts des inventeurs se sont portés sur deux familles de composés criblés et identifiés comme inhibiteurs de l'interaction PscE-PscG. Il s'agit des chélateurs de cations de type quinoline tel que le clioquinol et les sels de pyridinium tel que le sel de 3-alkyl pyridinium (3-APS). Le clioquinol fait partie des meilleurs inhibiteurs de l'interaction PscE-PscG lors du criblage primaire Quant aux 3-APS, ce sont des fragments élémentaires constitutifs de structures polymériques naturelles d'origine marine possédant de nombreuses propriétés biologiques

C'est aussi un objet de la présente invention que de fournir un procédé d'identification d'une molécule inhibitrice de la machinerie de virulence de *Pseudomonas aeruginosa* comprenant une étape d'évaluation de la capacité de ladite molécule à inhiber l'interaction PscE/PscG.

Le procédé d'identification selon la présente invention prévoit la mise en contact d'une molécule à tester avec les protéines PscE et PscG et à évaluer la capacité de ladite molécule à inhiber l'interaction entre ces deux protéines. Comme indiqué plus avant, toute méthode d'étude et d'évaluation de l'interaction protéine-protéine est applicable dans le cadre de la présente invention à partir du moment ou elle permet de déterminer un pourcentage d'inhibition de l'interaction PscE-PscG.

Dans un mode de réalisation particulier de l'invention, le procédé d'identification selon l'invention est caractérisé en ce que l'évaluation de la capacité de ladite molécule à inhiber l'interaction PscE/PscG est réalisée par une technique ELISA.

Ainsi un procédé d'identification selon la présente invention comprend les étapes suivantes :
a) Fournir un support sur lequel est fixée une des deux molécules PscE ou PscG,
b) Mettre en contact le support avec une solution contenant la molécule PscE ou PscG, la molécule en solution correspondant à celle non fixée au support,
c) Ajouter une solution contenant la molécule à tester
d) Laver le support
e) Ajouter un anticorps primaire dirigé contre la molécule non fixée au support, Laver le support
f) Ajouter un anticorps secondaire dirigé contre le premier anticorps et couplé à une enzyme,
g) Laver le support
h) Ajouter un substrat de l'enzyme générant lors de son hydrolyse par l'enzyme un signal,
i) Quantifier le signal
j) Evaluer la capacité d'inhibition de la molécule à tester par comparaison avec une gamme étalon.

C'est aussi un objet de la présente invention que de fournir un dispositif pour l'identification d'une molécule inhibitrice de la machinerie de virulence de *Pseudomonas aeruginosa* comprenant un support solide sur la surface duquel est fixée une des deux molécules PscE ou PscG.

Dans un mode de réalisation particulier, le dispositif selon la présente invention est caractérisé en ce que le support solide est une plaque de microtitration munie d'au moins un puit, la molécule étant fixée au support au niveau dudit puit. La fixation de la molécule, PscE ou PscG, peut être réalisée par liaison covalente ou préférentiellement par liaison de faible énergie, en particulier liaison hydrophobe, liaison hydrogène ou liaison de type Van der Walls, c'est-à-dire encore par adsorption.

Ainsi le dispositif selon l'invention est caractérisé en ce que la fixation de la molécule au support est réalisée par liaison faible.

Dans un mode de réalisation particulier du dispositif selon la présente invention la surface du support solide est hydrophobe afin d'assurer la liaison, de préférence par adsorption, de la molécule audit support.

La présente invention vise aussi un procédé de fabrication d'un dispositif selon la présente invention caractérisé en ce que :
a1. un support solide est mis en contact avec une solution de PscE ou de PscG afin d'assurer la fixation de PscE ou PscG sur la surface dudit support,
b1. la solution de l'étape a1 est éliminée du support,
c1. le support est optionnellement lavé et/ou séché, et
d1. le dispositif de criblage est récupéré.

Dans un mode de réalisation particulier, la fabrication d'un dispositif selon l'invention est caractérisée en ce que la fixation de PscE ou PscG sur la surface du support est obtenue via liaisons faibles de type hydrophobe, hydrogène ou de Van der Walls.

Dans un mode particulier, le procédé de fabrication selon la présente invention est caractérisé en ce que la surface du support solide présente un traitement de surface hydrophobe. Enfin dans un mode de réalisation particulier, le procédé de fabrication d'un dispositif selon l'invention est caractérisé en ce que le support solide est une plaque de microtitration comprenant au moins un puit.

A titre particulier, une plaque de microtitration de type NUNC est particulièrement adaptée.

C'est aussi un autre objet de la présente invention que de fournir l'utilisation d'un dispositif selon la présente invention pour l'identification de molécules aptes à inhiber la machine de virulence de *Pseudomonas aeruginosa.*

Enfin, la présente invention vise l'utilisation d'un dispositif selon la présente invention pour l'identification de molécules aptes à prévenir et/ou traiter une infection pathogène causée par *Pseudomonas aeruginosa.*

Il est décrit enfin au moins l'un des composés choisi parmi : l'acide pentétique, le clioquinol, le myricétine, le bensérazide, le gossypol, un sel de 3-alkylpyridium 6 (3-APS), et l'héderagénine pour son utilisation pour prévenir et/ou traiter une infection pathogène causée par *Pseudomonas aeruginosa.*

De manière préférentielle le composé selon l'invention pour son utilisation pour prévenir et/ou traiter une infection pathogène causée par *Pseudomonas aeruginosa* est caractérisé en ce que l'infection est une infection nosocomiale.

Dans un mode de réalisation particulier, l'infection pathogène est une infection choisie parmi les infections des yeux, les infections des plaies, en particulier suite à une brûlure et/ou une opération chirurgicale, les infections des voies urinaires, en particulier de la vessie ou de l'urètre par exemple après sondage, les infections du système gastro-intestinal, les infections d'un poumon en particulier après bronchoscopie, les méningites, en particulier d'inoculation, et les septicémies.

### Exemple 1 : Réalisation du test ELISA

50 µL de protéine PscG à 1 µM (pour le criblage de librairies) ou 2.2 µM (pour le criblage de molécules nouvellement synthétisées) est incubé dans une plaque NUNC 96 puits pendant 8 h à 4°C. Les puits sont lavés trois fois avec 200 µL de 1X PBS/0.1% Tween20®. Les puits sont traités toute une nuit à 4°C avec 200 µL d'une solution de lait à 5% dans 1X PBS/0.1% Tween20®. La solution est ensuite jetée et la plaque séchée par égouttage.

Le test est réalisé comme suit :
- ***Contrôle négatif (plus faible niveau de signal):*** 100 µL de tampon (25 mM Tris-HCl, 250 mM NaCl, pH 8.0);
- ***Contrôle positif (plus fort niveau de signal):*** 50 µL de tampon et 50 µL d'une solution à tester à 25 µM (criblage de librairie) ou 3 µM (criblage de molécules analogues) de la protéine PscE;
- ***Test des inhibiteurs:*** 40 µL tampon, 50 µL d'une solution à 25 µM (criblage de librairie) ou 3 µM (criblage de molécules analogues) de protéine PscE et 10 µL de la solution contenant l'inhibiteur à tester.

Les solutions sont laissées pendant 4h à température ambiante. Les puits sont lavés trois fois avec 200 µL de 1X PBS/0.1% Tween20®. L'anticorps anti-PscE dilué à 1:2,000 dans 1X PBS/0.1% Tween20®, 50 µL, est ajouté et le tout est laisse 2 h à température ambiante. Les puits sont laves trois fois avec 200 µL de 1X PBS/0.1% Tween20®. L'anticorps anti souris conjugué avec HRP est dilué à 1:5,000 dans 1X PBS/0.1% Tween20® et 50 µL sont ajoutés et laissés pendant 2h à température ambiante. Les puits sont lavés trois fois avec 200 µL of 1X PBS/0.1% Tween20®.

La révélation du test ELISA est réalisée par ajout de 50 µL de réactif TMB suivi par une incubation de 15 minutes à température ambiante dans l'obscurité. L'arrêt de la réaction est réalisé par ajout de 50 µL of 2N HCl. La plaque est lue à t 450 nm.

### Résultats d'inhibition

**Tableau 1. Molécules identifiées dans le criblage primaire PscE-PscG à 50 µM**

| Molécules | Nom | Propriétés connues | % inhibition PscE-PscG à 50 µM |
|---|---|---|---|
| 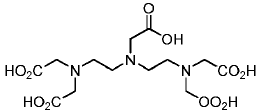 | Acide Pentétique | | 94% |
| 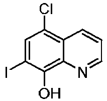 | Clioquinol | Antifongique Antiprotozoa ire AntiAlzheimer | 93.7% (IC₅₀ = 5 µM) |
| 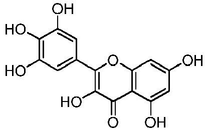 | Myricétine | Anti-oxidant | 80.5% |
| 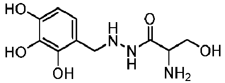 | Bensérazide | Inhibiteur de décarboxylase | 86.3% (IC₅₀ = 11 µM) |
| 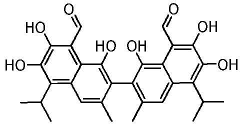 | Gossypol | | 73.9% |
| 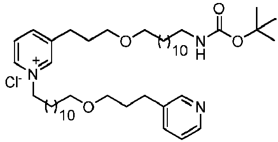 | Sels de 3-alkyl pyridium **6** (3-APS) | | 56% |
| 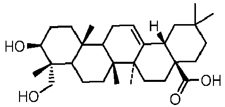 | Héderagénine | | 58% |

### Exemple 2 Synthèse modulable des 3-APS et des hybrides 3-APS/clioquinol et essais d'inhibition de l'intéraction PscE-PscG

De nouveaux analogues de 3-APS avec différentes longueurs de chaîne, avec la possibilité de réaliser des molécules « hybrides » 3-APS/clioquinol pour potentialiser les deux clusters par effet synergique (Schéma 1). L'approche synthétique de l'invention repose sur la réaction de Zincke. Le procédé de l'invention concerne une stratégie de synthèse combinatoire qui permet à partir de seulement n fragments (dans l'exemple reporté, n = 5 fragments, i.e. **12a-e)** + l'analogue du clioquinol **8,** de pouvoir obtenir potentiellement 2ⁿ × 2 nouveaux analogues en très peu d'étapes (3 à 4 étapes). Une réaction de Sharpless-Meldal^{[43,44]} (réaction « click ») en présence de K₂CO₃ permet de former **16** en une seule étape sans déprotection préalable du groupement silylé sur **8.**

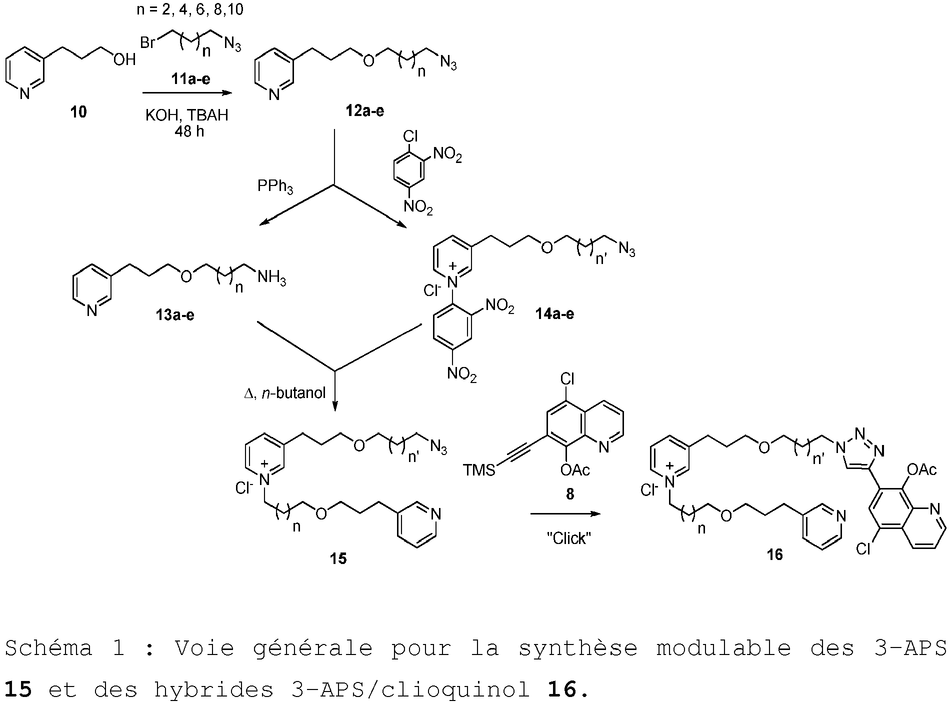

### Résultats

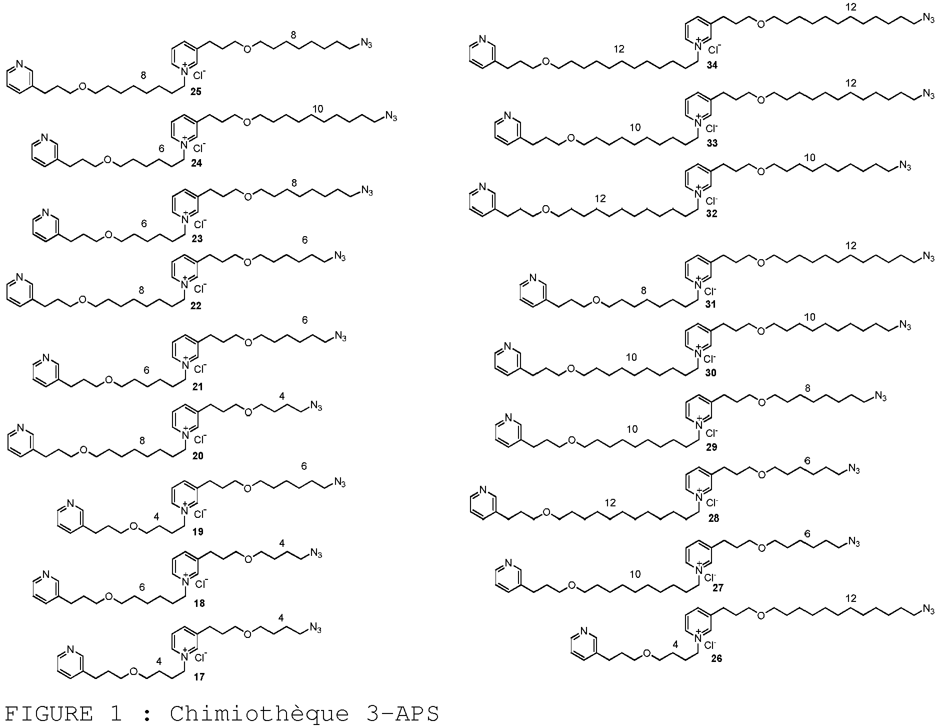

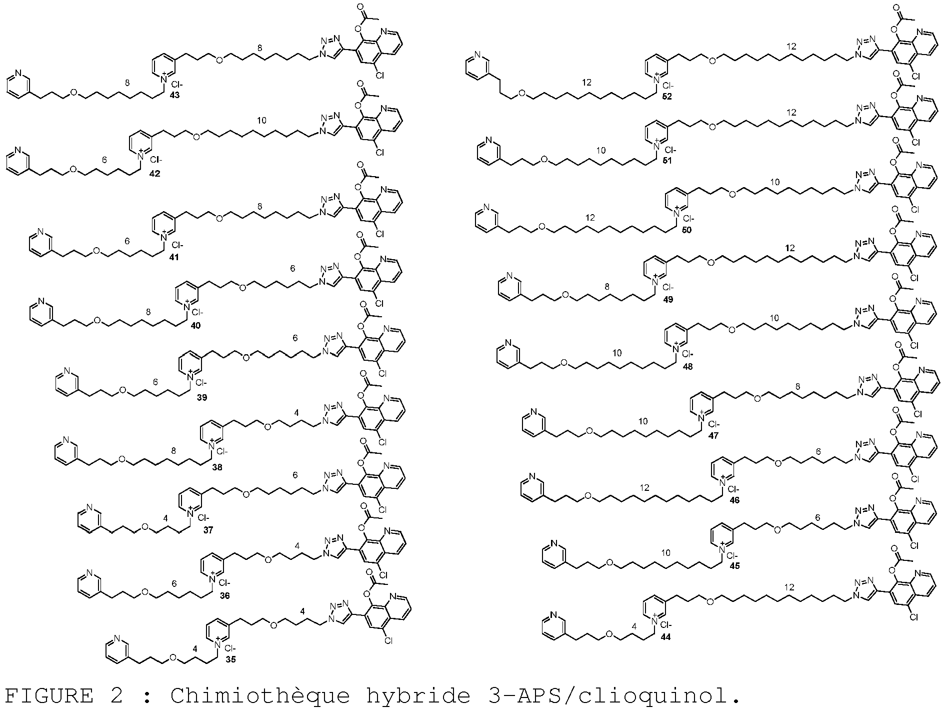

13 Analogues 3-APS et les 18 analogues 3-APS/clioquinol ont été évalués sur le test ELISA (Tableau 4). Sur les 31 molécules testées, quatre molécules **(23, 30, 31** et **33)** en série 3-APS et huit molécules **(36, 37, 39, 40, 41, 45, 48** et **49)** en série hybride 3-APS/clioquinol ont démontré un effet inhibiteur supérieur au clioquinol.

**Tableau 2 : Résultat sur test ELISA des nouveaux analogues synthétiques**

| Cluster | molécule | PscE-PscG à 50 µM |
|---|---|---|
| référence | clioquinol | 45.2% |
| 3-APS | **17** | pas d'inhibition |
| | **18** | pas d'inhibition |
| | **19** | 19.8% |
| | **20** | pas d'inhibition |
| | **22** | 88.8% |
| | **24** | 38.6% |
| | **26** | 44.2% |
| | **27** | 39.6% |
| | **28** | 37.8% |
| | **29** | 24% |
| | **30** | 52.8% |
| | **31** | 82.9% |
| | **33** | 54.2% |
| | **34** | 30.6% |
| 3-APS/clioquinol | **35** | 29.8% |
| | **36** | 54.2% |
| | **37** | 92.5% |
| | **38** | 27.3% |
| | **39** | 97.3% |
| | **40** | 87.2% |
| | **41** | 69.1% |
| | **42** | pas d'inhibition |
| | **43** | 29.5% |
| | **44** | 33.4% |
| | **45** | 71.6% |
| | **46** | pas d'inhibition |
| | **47** | pas d'inhibition |
| | **48** | 72.3% |
| | **49** | 69.8% |
| | **50** | 18.9% |
| | **51** | 28.9% |
| | **52** | 3.4% |

### EXAMPLE 3 : Préparation des molécules

Pour 1-azido-4-butane **11a:** Coutrot, F., and Busseron, E., Controlling the Chair Conformation of a Mannopyranose in a Large-Amplitude [2]Rotaxane Molecular Machine, Chem.-Eur. J. 2009, 15, 5186-5190

Pour 1-azido-6-hexane **11 b** et 1-azido-8-octane **11c:** Agnew, H. D., Rohde, R. D., Millward, S. W., Nag, A., Yeo, W.-S., Hein, J. E., Pitram, S. M., Tariq, A. A., Burns, V. M., Krom, R. J., Fokin, V. V., Sharpless, K. B., and Heath, J. R., Iterative In Situ Click Chemistry Creates Antibody-like Protein-Capture Agents, Angew. Chem., Int. Ed. 2009, 48, 4944-4948.

### Procédure générale: synthèse de 1-azido-10-bromodecane (11d)

Une solution de 1,10-dibromodecane (9.23 g, 30.7 mmol) et NaN₃ (2 g, 30,7 mmol) dans le DMF (35 mL) est agitée à température ambiante pendant 48 h. De l'éther est ajouté (150 mL) et la phase organique est lavée à l'eau (3×150 mL). La phase organique est séchée et évaporée (MgSO₄). Le produit est purifié par flash chromatographie (2% CH₂Cl₂/cyclohexane, gel de silice) pour donner **11d** sous la forme d'une huile incolore (4.27 g, 53%).

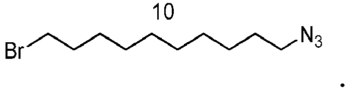

R*_{f}* = 0.38 (cyclohexane/EtOAc 98:2); ¹H NMR (400 MHz, CDCl₃) *δ* 1.30-1.40 (m, 10H), 1.40-1.44 (m, 2H), 1.59 (m, 2H), 1.85 (m, 2H), 3.25 (t, *J* = 7.0 Hz, 2H), 3.40 (t, *J* = 6.9 Hz, 2H); ¹³C NMR (100 MHz, CDCl₃) *δ* 26.8 (CH₂), 28.3 (CH₂), 28.8 (CH₂), 28.9 (CH₂), 29.2 (CH₂), 29.4 (CH₂), 29.5 (CH₂), 32.9 (CH₂), 34.1 (CH₂), 51.6 (CH₂).

### 1-azido-12-bromododecane (11e)

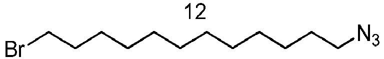

A partir de 1,12-dibromododecane (5.05 g, 15.4 mmol), le produit **11e** (2.2 g, 49%) est obtenu par flash chromatographie (silica gel, 2% CH₂Cl₂/cyclohexane) sous la forme d'une huile incolore; R*_{f}* = 0.5 (2% CH₂Cl₂/cyclohexane); ¹H NMR (400 MHz, CDCl₃) *δ* 1,25-1.33 (m, 12H), 1.59 (m, 1H), 1.85 (m, 1H), 3.25 (t, *J* = 6.9 Hz, 1H), 3.40 (t, *J* = 6.9 Hz, 1H); ¹³C NMR (100 MHz, CDCl₃) *δ* 26.9 (CH₂), 28.3 (CH₂), 28.9 (CH₂), 29.0 (CH₂), 29.3 (CH₂), 29.5 (CH₂), 29.6 (3xCH₂), 33.0 (CH₂), 34.2 (CH₂), 51.6 (CH₂).

### Procédure générale de synthèse de 3-(3-(4-azidobutoxy)propyl)pyridine (12a)

A un mélange de 1-azido-4-bromobutane (1.29 g, 7.2 mmol) et de 3-(3-hydroxypropyl)-pyridine (1.04 g, 7.6 mmol) est ajouté du KOH (730 mg, 13.0 mmol) et du tétrabutylammonium hydrogène sulphate (123 mg, 0,36 mmol). Après mélange sous argon pendant 48 h, du CH₂Cl₂ est ajouté et le mélange est filtré. Le solvant est ôté sous vide et le produit purifié par flash chromatographie (30% EtOAc/cyclohexane, gel de silice) pour donner **12a** sous la forme d'une huile jaune (682 mg, 40%). 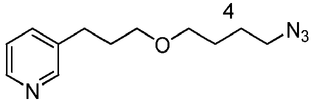 C₁₂H₁₈N₄O
M=234,15g/mol

R*_{f}* = 0.13 (20% EtOAc/cyclohexane) ; ¹H NMR (400 MHz, CDCl₃) *δ* 1.57-1.75 (m, 4H), 1.89 (m, 2H), 2.70 (t, *J* = 7.5 Hz, 2H), 3.31 (t, *J* = 6.6 Hz, 2H), 3.42 (t, *J* = 6.3 Hz, 2H), 3.44 (t, *J* = 6.2 Hz, 2H), 7.20 (dd, *J* = 7.7, 4.8 Hz, 1H), 7.51 (d, *J =* 7.7 Hz, 1H), 8.43-8.45 (m, 2H); ¹³C NMR (100 MHz, CDCl₃) *δ* 26.0 (CH₂), 27.0 (CH₂), 29.6 (CH₂), 31.1 (CH₂), 51.4 (CH₂), 69.7 (CH₂), 70.3 (CH₂), 123.4 (CH), 136.0 (CH), 137.3 (C), 147.4 (CH), 150.0 (CH); LRMS (ESI+) *m*/*z* (%): 357 (70) [*M*+Na]⁺; 235 (100) [*M*+H]⁺.

### 3-(3-(6-azidohexyloxy)propyl)pyridine (12b)

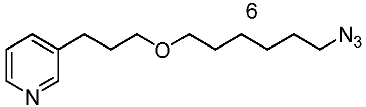

C₁₄H₂₂N₄O
M=262,18 g/mol

A partir 1-azido-6-bromohexane (2.8 g, 13.4 mmol), le produit **12b** (2.56 g, 72%) est obtenu sous la forme d'une huile jaune.

R*_{f}* = 0.14 (20% EtOAc/cyclohexane); ¹H NMR (400 MHz, CDCl₃) *δ* 1.39-1.42 (m, 4H), 1.60-1.63 (m, 4H), 1.86-1.98 (m, 2H), 2.70 (t, *J* = 7,5 Hz, 2H), 3.27 (t, *J* = 6.9 Hz, 2H), 3.35-3.43 (m, 4H), 7.21 (dd, *J* = 7.7, 4.8 Hz, 1H), 7.51 (dd, *J* = 7.7, 1.6 Hz, 1H), 8,44-8,47 (m, 2H); ¹³C NMR (100 MHz, CDCl₃) *δ* 25.9 (CH₂), 26.7 (CH₂), 28.9 (CH₂), 29.6 (CH₂), 29.7 (CH₂), 31.1 (CH₂), 51.5 (CH₂), 69.6 (CH₂), 70.9 (CH₂), 123.4 (CH), 136.0 (CH), 137.3 (C), 147.4 (CH), 150.1 (CH); LRMS (ESI+) *m*/*z* (%): 285 (30) [*M*+Na]⁺; 263 (100) [*M*+H]⁺.

### 3-(3-(8-azidooctyloxy)propyl)pyridine (12c)

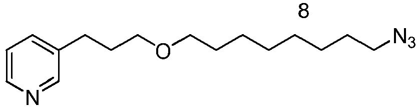

FW = 290,21g/mol
C₁₆H₂₆N₄O

A partir de 1-azido-8-bromo-octane (2.68 g, 11.4 mmol), le produit **12c** (2.54 g, 76.5%) est obtenu sous la forme d'une huile jaune.

R*_{f}* = 0.18 (20% EtOAc/cyclohexane); ¹H NMR (400 MHz, CDCl₃) *δ* 1.34-1.41 (m, 8H), 1.54-1.63 (m, 4H), 1.89 (m, 2H), 2.70 (t, *J* = 7,4 Hz, 2H), 3.25 (t, *J* = 6.9 Hz, 2H), 3.40 (t, *J* = 6.6 Hz, 2H), 3.41 (t, *J* = 6.3 Hz, 2H), 7.21 (dd, *J* = 7.7, 4.8 Hz, 1H), 7.51 (d, *J* = 7.7 Hz, 1H), 8,44 (dd, *J* = 4.8, 1.6 Hz, 1H), 8.46 (d, *J* = 1.6 Hz, 1H); ¹³C NMR (100 MHz, CDCl₃) *δ* 26.2 (CH₂), 26.8 (CH₂), 28.9 (CH₂), 29.2 (CH₂), 29.4 (CH₂), 29.6 (CH₂), 29.8 (CH₂), 31.1 (CH₂), 51.6 (CH₂), 69.6 (CH₂), 71.1 (CH₂), 123.4 (CH), 136.0 (CH), 137.3 (C), 147.4 (CH), 150.1 (CH); LRMS (ESI+) *m*/*z* (%): 313 (72) [*M*+Na]⁺; 291 (100) [*M*+H]⁺

### 3-(3-(10-azidodecyloxy)propyl)pyridine (12d)

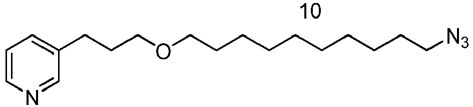

C₁₈H₃₀N₄O
M=318,24 g/mol

A partir de 1-azido-10-bromo-decane (3.52 g, 13.4 mmol), le produit **12d** (3.42 g, 80%) est obtenu sous la forme d'une huile jaune.

R*_{f}* = 0.21 (20% EtOAc/cyclohexane); ¹H NMR (400 MHz, CDCl₃) *δ* 1.23-1.34 (m, 12H), 1.54-1.64 (m, 4H), 1.89 (m, 2H), 2.70 (t, *J* = 7,5 Hz, 2H), 3.25 (t, *J* = 7.0 Hz, 2H), 3.38-3.43 (m, 4H), 7.20 (dd, *J* = 7.5, 4.8 Hz, 1H), 7.51 (d, *J* = 7.5 Hz, 1H), 8,44 (dd, *J* = 4.8, 1.6 Hz, 1H), 8.46 (d, *J* = 1.6 Hz, 1H); ¹³C NMR (100 MHz, CDCl₃) *δ* 26.4 (CH₂), 26.8 (CH₂), 29.0 (CH₂), 29.3 (CH₂), 29.5 (CH₂), 29.6 (CH₂), 29.6 (CH₂), 29.7 (CH₂), 29.9 (CH₂), 31.2 (CH₂), 51.6 (CH₂), 69.6 (CH₂), 71.2 (CH₂), 123.4 (CH), 136.0 (CH), 137.4 (C), 147.5 (CH), 150.2 (CH); LRMS (ESI+) *m*/*z* (%): 341 (55) [*M*+Na]⁺; 319 (100) [*M*+H]⁺.

### 3-(3-(12-azidododecyloxy)propyl)pyridine (12e)

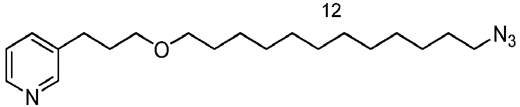

C₂₀H₃₄N₄O
MM = 346

A partir de 1-azido-12-bromo-dodecane (3.71 g, 12.8 mmol), le produit **12e** (3.34 g, 75%) est obtenu sous la forme d'une huile jaune.

R*_{f}* = 0.21 (20% EtOAc/cyclohexane); ¹H NMR (400 MHz, CDCl₃) *δ* 1.23-1.40 (m, 14H), 1.54-1.63 (m, 4H), 1.89 (m, 2H), 2.71 (m, 2H), 3.25 (t, *J* = 7.0 Hz, 2H), 3.40 (t, J = 6.7 Hz, 2H), 3.41 (f, *J* = 6.2 Hz, 2H), 7.21 (dd, *J* = 7.8, 4.8 Hz, 1H), 7.51 (dt, *J* = 7.8, 1.7 Hz, 1H), 8,44 (dd, *J* = 4.8, 1.7 Hz, 1H), 8.46 (d, *J* = 1.7 Hz, 1H); ¹³C NMR (100 MHz, CDCl₃) *δ* 26.0 (CH₂), 26.5 (CH₂), 28.9 (CH₂), 29.2 (5×CH₂), 29.3 (2×CH₂), 29.6 (CH₂), 30.8 (CH₂), 51.2 (CH₂), 69.1 (CH₂), 70.8 (CH₂), 123.0 (CH), 135.6 (CH), 137.0 (C), 147.0 (CH), 149.8 (CH); LRMS (ESI+) *m*/*z* (%): 347 (100) [*M*+H]⁺, 319 (5).

### Procédure générale de synthèse de 3-(3-(4-azidobutoxy)propyl)pyridine (13a)

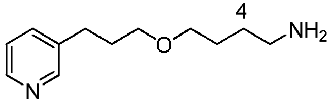

C₁₂H₂₀N₂O
M=208,16g/mol

A une solution d'azide **12a** (390 mg, 1.67 mmol) dans la pyridine (12 mL) est ajoutée une portion de PPh₃ (828 mg, 3.2 mmol) à 0°C. Après 30 min à cette température, le mélange est agité toute la nuit à température ambiante. Après refroidissement à 0°C, une solution concentrée d'ammoniac aqueuse est ajoutée (0.91 mL) et le mélange est agité pendant une nuit à température ambiante. La pyridine est ôtée et une solution de HCl 1N (19 mL) est ajoutée. La solution est lavée à l'éther. Du Na₂CO₃ solide (2.4 g) est ajouté par portion et le produit est extrait avec CH₂Cl₂. Le solvant organique est séché (MgSO₄) et évaporé sous vide. Le produit est purifié par flash chromatographie (10 to 15% MeOH/CH₂Cl₂ et 15% MeOH/1%Et₃N/CH₂Cl₂) pour obtenir le produit **13a** (212 mg, 58%) sous la forme d'une huile.

R*_{f}* = 0.05 (10% MeOH/CH₂Cl₂); ); ¹H NMR (400 MHz, CDCl₃) *δ* 1.43 (m, 2H), 1.52 (m, 2H), 1.80 (m, 2H), 2.58-2.67 (m, 4H), 3.30-3.35 (m, 4H), 7.12 (dd, *J* = 7.8, 4.8 Hz, 1H), 7.43 (d, *J* = 7.8 Hz, 1H), 8,35 (dd, *J* = 4.8, 1.6 Hz, 1H), 8.37 (d, *J* = 1.6 Hz, 1H); ¹³C NMR (100 MHz, CDCl₃) *δ* 27.0 (CH₂), 29.4 (CH₂), 30.3 (CH₂), 30.9 (CH₂), 41.9 (CH₂), 69.3 (CH₂), 70.7 (CH₂), 123.2 (CH), 135.8 (CH), 137.1 (C), 147.2 (CH), 149.9 (CH); LRMS (ESI+) *m*/*z* (%): 231 (4) [*M*+Na]⁺ ; 209 (100) [*M*+H]⁺.

### 10-(3-(pyridin-3-yl)propoxy)hexan-1-amine (13b)

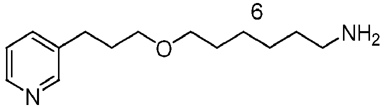

C₁₄H₂₄N₂O
M=236,19g/mol

R*_{f}* = 0.08 (15% MeOH/CH₂Cl₂); ¹H NMR (400 MHz, CDCl₃) *δ* 1.30-1.41 (m, 4H), 1.46 (m, 2H), 1.91 (m, 2H), 2.68-2.72 (m, 4H), 3.40 (t, *J* = 6.6 Hz, 2H), 3.41 (t, *J* = 6.3 Hz, 2H), 7.21 (dd, *J* = 7.7, 4.8 Hz, 1H), 7.51 (d, *J* = 7.7 Hz, 1H), 8,44 (dd, *J* = 4.8, 1.5 Hz, 1H), 8.46 (d, *J* = 1.5 Hz, 1H); ¹³C NMR (100 MHz, CDCl₃) *δ* 26.3 (CH₂), 26.9 (CH₂), 29.7 (CH₂), 29.9 (CH₂), 31.2 (CH₂), 33.9 (CH₂), 42.2 (CH₂), 69.5 (CH₂), 71.0 (CH₂), 123.3 (CH), 135.9 (CH), 137.2 (C), 147.4 (CH), 150.1 (CH); LRMS (ESI+) *m*/*z* (%): 259 (12) [*M*+Na]⁺ ; 237 (100) [*M*+H]⁺.

### 8-(3-(pyridin-3-yl)propoxy)octan-1-amine (13c)

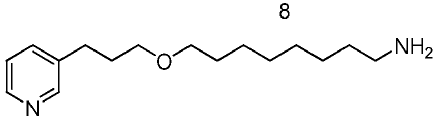

C₁₆H₂₈N₂O
M=264,22g/mol

A partir de **12c** (2.0 g, 6.89 mmol), le produit **13c** (1.32 g, 73%) est obtenu sous la forme d'une huile incolore.

R*_{f}* = 0.05 (10% MeOH/CH₂Cl₂); ¹H NMR (400 MHz, CDCl₃) *δ* 1.30-1.43 (m, 8H), 1.46 (m, 2H), 1.57 (m, 2H), 1.90 (m, 2H), 2.69-2.72 (m, 4H), 3.38-3.43 (m, 4H), 7.21 (dd, *J* = 7.7, 4.8 Hz, 1H), 7.51 (d, *J* = 7.7 Hz, 1H), 8.44 (dd, *J* = 4.8, 1.5 Hz, 1H), 8.46 (d, *J* = 1.5 Hz, 1H); ¹³C NMR (100 MHz, CDCl₃) *δ* 26.3 (CH₂), 27.0 (CH₂), 29.6 (CH₂), 29.7 (CH₂), 29.9 (CH₂), 31.2 (CH₂), 33.7 (CH₂), 42.3 (CH₂), 69.6 (CH₂), 71.3 (CH₂), 123.5 (CH), 136.1 (CH), 137.4 (C), 147.5 (CH), 150.2 (CH); LRMS (ESI+) *m*/*z* (%): 287 (7) [*M*+Na]⁺ ; 265 (100) [*M*+H]⁺.

### 10-(3-(pyridin-3-yl)propoxy)decan-1-amine (13d)

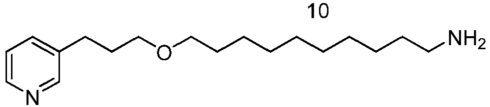

C₁₈H₃₂N₂O
M=292,25g/mol

A partir de **12d** (2.0 g, 6.29 mmol), le produit **13d** (1.58 g, 95%) est obtenu sous la forme d'une huile incolore.

R*_{f}* = 0.10 (10% MeOH/CH₂Cl₂); ¹H NMR (400 MHz, CDCl₃) *δ* 1.26-1.39 (m, 12H), 1.47 (m, 2H), 1.57 (m, 2H), 1.89 (m, 2H), 2.67-2.72 (m, 4H), 3.38-3.43 (m, 4H), 7.21 (dd, *J* = 7.7, 4.8 Hz, 1H), 7.51 (d, *J* = 7.7 Hz, 1H), 8.44 (dd, *J* = 4.8, 1.5 Hz, 1H), 8.46 (d, *J* = 1.5 Hz, 1H); ¹³C NMR (100 MHz, CDCl₃) *δ* 26.4 (CH₂), 27.0 (CH₂), 29.6 (CH₂), 29.7 (CH₂), 29.7 (CH₂), 29.7 (CH₂), 29.7 (CH₂), 29.7 (CH₂), 29.9 (CH₂), 31.2 (CH₂), 33.4 (CH₂), 42.2 (CH₂), 69.6 (CH₂), 71.3 (CH₂), 123.5 (CH), 136.1 (CH), 137.4 (C), 147.5 (CH), 150.2 (CH); LRMS (ESI+) *m*/*z* (%): 315 (4) [*M*+Na]⁺; 293 (100) [*M*+H]⁺.

### 12-(3-(pyridin-3-yl)propoxy)dodecan-1-amine (13e)

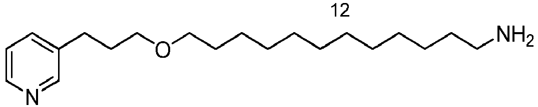

C₂₀H₃₆N₂O
MM = 320

A partir de **12e** (707 mg, 2.04 mmol), le produit **13e** (370 mg, 47%) est obtenu sous la forme d'une huile incolore.

R*_{f}* = 0.13 (20% MeOH/CH₂Cl₂); ¹H NMR (400 MHz, CDCl₃) *δ* 1.15-1.28 (m, 16H), 1.35 (m, 2H), 1.48 (m, 2H), 1.80 (m, 2H), 2.55-2.65 (m, 4H), 3.28-3.35 (m, 4H), 7.11 (dd, *J* = 7.7, 4.8 Hz, 1H), 7.42 (d, *J* = 7.7 Hz, 1H), 8.34 (dd, *J* = 4.8, 1.5 Hz, 1H), 8.37 (d, *J* = 1.5 Hz, 1H); ¹³C NMR (100 MHz, CDCl₃) *δ* 26.1 (CH₂), 26.7 (CH₂), 29.3 (5×CH₂), 29.4 (3×CH₂), 29.6 (CH₂), 30.9 (CH₂), 41.9 (CH₂), 69.2 (CH₂), 70.9 (CH₂), 123.1 (CH), 135.7 (CH), 137.1 (C), 147.1 (CH), 149.8 (CH); LRMS (ESI+) *m*/*z* (%): 343 (3) [*M*+Na]⁺ ; 321 (100) [*M*+H]⁺.

### Procédure générale de synthèse de 3-(3-(4-azidobutoxy)propyl)-1-(2,4-dinitrophenyl)pyridinium chloride (14a)

Le produit **12a** (521 mg, 2.2 mmol) et le 1-chloro-2,4-dinitrobenzene (897 mg, 4.4 mmol) dans l'ethanol (13 mL) est chauffé à 135°C sous agitation pendant 48 h. L'éthanol est enlevé sous vide et le produit purifié par flash chromatographie (10% to 15% methanol/CH₂Cl₂, gel de silice) pour donner **14a** (920 mg, 86%) sous la forme d'une huile brune. 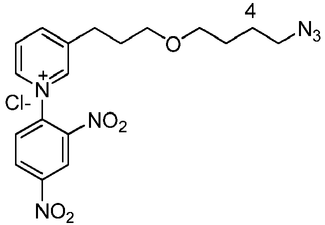 C₁₈H₂₁ClN₆O₅
MM = 436.5

R*_{f}* = 0.08 (10% MeOH/CH₂Cl₂); ¹H NMR (400 MHz, CDCl₃) *δ* 1.60-1.68 (m, 4H), 2.09 (m, 2H), 3.12 (t, *J* = 7.6 Hz, 2H), 3.33 (m, 2H), 3.49 (m, 2H), 3.55 (t, *J* = 6.0 Hz, 2H), 8.34 (d, *J* = 8.2, 6.3 Hz, 1H), 8.38 (d, *J* = 8.7 Hz, 1H), 8.88 (d, *J* = 8.7 Hz, 1H), 8.93 (dd, *J* = 8.7, 2.5 Hz, 1H), 9.23 (d, *J* = 6.3 Hz, 1H), 9.25 (d, *J* = 2.5 Hz, 1H), 9.33 (s, 1H); ¹³C NMR (100 MHz, CDCl₃) *δ* 26.9 (CH₂), 28.0 (CH₂), 30.7 (CH₂), 31.2 (CH₂), 52.4 (CH₂), 70.3 (CH₂), 71.4 (CH₂), 123.3 (CH), 129.1 (CH), 131.4 (CH), 132.9 (CH), 140.2 (C), 144.6 (C), 144.8 (CH), 145.5 (C), 146.5 (CH), 150.2 (CH), 151.1 (C); LRMS (ESI+) *m*/*z* (%): 401 (100) [*M*]⁺.

### 3-(3-(6-azidohexyloxy)propyl)-1-(2,4-dinitrophenyl)pyridinium chloride (14b)

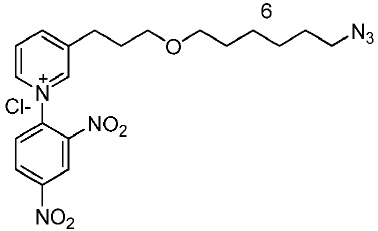

C₂₀H₂₅ClN₆O₅
M=464.5 g/mol

A partir de **12b** (1.8 g, 6.89 mmol), le produit **14b** (2.99 g, 93.5%) est obtenu sous la forme d'une huile brune.

R*_{f}* = 0.09 (10% MeOH/CH₂Cl₂); ¹H NMR (400 MHz, CDCl₃) *δ* 1.35-1.45 (m, 4H), 1.53-1.62 (m, 4H), 2.07 (m, 2H), 3.10 (t, *J* = 7.6 Hz, 2H), 3.29 (*J* = 6.8 Hz, 2H), 3.45 (*J* = 6.5 Hz, 2H), 3.53 (t, *J* = 6.0 Hz, 2H), 8.33 (d, *J* = 8.0, 6.3 Hz, 1H), 8.37 (d, *J* = 8.7 Hz, 1H), 8.86 (d, *J* = 8.0 Hz, 1H), 8.92 (dd, *J* = 8.6, 2.4 Hz, 1H), 9.22 (d, *J* = 6.3 Hz, 1H), 9.25 (d, *J* = 2.4 Hz, 1H), 9.32 (s, 1H); ¹³C NMR (100 MHz, CDCl₃) *δ* 26.9 (CH₂), 27.7 (CH₂), 29.8 (CH₂), 30.7 (2×CH₂), 31.3 (CH₂), 52.5 (CH₂), 70.3 (CH₂), 72.0 (CH₂), 123.3 (CH), 129.1 (CH), 131.3 (CH), 132.9 (CH), 140.2 (C), 144.6 (C), 144.8 (C), 145.5 (C), 146.6 (CH), 150.2 (CH), 151.1 (C); LRMS (ESI+) *m*/*z* (%): 429 (100) [*M*]⁺.

### 3-(3-(8-azidooctyloxy)propyl)-1-(2,4-dinitrophenyl)pyridinium chloride (14c)

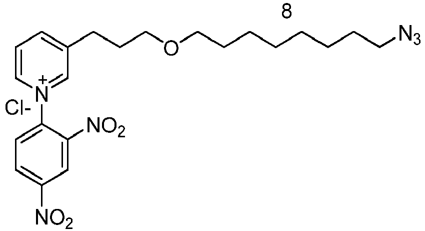

C₂₂H₂₉ClN₆O₅
M=492,2g/mol

A partir de **12c** (1.9 g, 6.89 mmol), le produit **14c** (2.88 g, 89%) est obtenu sous la forme d'une huile brune.

R*_{f}* = 0.09 (10% MeOH/CH₂Cl₂); ¹H NMR (400 MHz, CDCl₃) *δ* 1.35-1.45 (m, 8H), 1.53-1.63 (m, 4H), 2.09 (m, 2H), 3.12 (t, *J* = 7.6 Hz, 2H), 3.30 (*J* = 6.8 Hz, 2H), 3.46 (*J* = 6.6 Hz, 2H), 3.54 (t, *J* = 6.0 Hz, 2H), 8.35 (d, *J* = 7.9, 6.3 Hz, 1H), 8.39 (d, *J* = 8.7 Hz, 1H), 8.88 (d, *J* = 7.9 Hz, 1H), 8.93 (dd, *J* = 8.6, 2.4 Hz, 1H), 9.19 (d, *J* = 6.3 Hz, 1H), 9.26 (m, 2H); ¹³C NMR (100 MHz, CDCl₃) *δ* 27.3 (CH₂), 27.9 (CH₂), 30.0 (CH₂), 30.3 (CH₂), 30.6 (CH₂), 30.8 (CH₂), 30.9 (CH₂), 31.3 (CH₂), 52.6 (CH₂), 70.3 (CH₂), 72.2 (CH₂), 123.3 (CH), 129.1 (CH), 131.3 (CH), 132.8 (CH), 140.3 (C), 144.7 (C), 144.8 (CH), 145.6 (C), 146.6 (CH), 150.3 (CH), 151.3 (C); LRMS (ESI+) *m*/*z* (%): 457 (100) [*M*]⁺.

### 3-(3-(10-azidodecyloxy)propyl)-1-(2,4-dinitrophenyl)pyridinium chloride (14d)

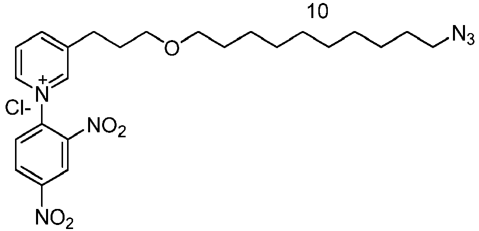

C₂₄H₃₃ClN₆O₅
M = 520.5 g/mol

A partir de **12d** (2.42 g, 7.6 mmol), le produit **14d** (3.5 g, 88.5%) est obtenu sous la forme d'une huile brune.

Rf = 0.15 (10% MeOH/CH₂Cl₂); ¹H NMR (400 MHz, CDCl₃) *δ* 1.30-1.40 (m, 12H), 1.52-1.62 (m, 4H), 2.07 (m, 2H), 3.09 (t, *J* = 7.6 Hz, 2H), 3.27 (*J* = 6.9 Hz, 2H), 3.44 (*J* = 6.6 Hz, 2H), 3.52 (t, *J* = 6.0 Hz, 2H), 8.31 (d, *J* = 8.0, 6.3 Hz, 1H), 8.36 (d, *J* = 8.7 Hz, 1H), 8.83 (d, *J* = 8.0 Hz, 1H), 8.91 (dd, *J* = 8.6, 2.4 Hz, 1H), 9.21 (d, *J* = 6.3 Hz, 1H), 9.24 (d, *J* = 2.4 Hz, 1H), 9.31 (s, 1H); ¹³C NMR (100 MHz, CDCl₃) *δ* 27.4 (CH₂), 27.9 (CH₂), 30.0 (CH₂), 30.3 (CH₂), 30.7 (4×CH₂), 30.9 (CH₂), 31.3 (CH₂), 52.5 (CH₂), 70.3 (CH₂), 72.2 (CH₂), 123.2 (CH), 129.1 (CH), 131.3 (CH), 132.9 (CH), 140.2 (C), 144.6 (C), 144.8 (CH), 145.5 (C), 146.5 (CH), 150.2 (CH), 151.1 (C); LRMS (ESI+) *m*/*z* (%): 485 (100) [*M*]⁺

### 3-(3-(12-azidododecyloxy)propyl)-1-(2,4-dinitrophenyl)pyridinium chloride (14e)

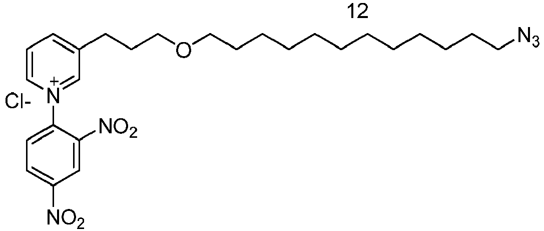

C₂₆H₃₇ClN₆O₅
MM = 548.5

A partir de **12e** (1.95 g, 5.64 mmol), le produit **14e** (2.85 g, 91%) est obtenu sous la forme d'une huile brune.

Rf = 0.17 (10% MeOH/CH₂Cl₂); ¹H NMR (400 MHz, CDCl₃) *δ* 1.28-1.40 (m, 16H), 1.51-1.62 (m, 4H), 2.06 (m, 2H), 3.09 (t, *J* = 7.6 Hz, 2H), 3.28 (*J* = 6.8 Hz, 2H), 3.43 (*J* = 6.8 Hz, 2H), 3.51 (t, *J* = 6.0 Hz, 2H), 8.31 (d, *J* = 8.1, 6.3 Hz, 1H), 8.35 (d, *J* = 8.7 Hz, 1H), 8.84 (d, *J* = 8.1 Hz, 1H), 8.91 (dd, *J* = 8.7, 2.4 Hz, 1H), 9.19 (d, *J* = 6.3 Hz, 1H), 9.25 (d, *J* = 2.4 Hz, 1H), 9.29 (s, 1H); ¹³C NMR (100 MHz, CDCl₃) *δ* 27.4 (CH₂), 27.9 (CH₂), 30.0 (CH₂), 30.4 (CH₂), 30.7 (4×CH₂), 30.8 (2×CH₂), 30.9 (CH₂), 31.3 (CH₂), 52.6 (CH₂), 70.3 (CH₂), 72.2 (CH₂), 123.2 (CH), 129.1 (CH), 131.3 (CH), 132.9 (CH), 140.3 (C), 144.7 (C), 144.8 (CH), 145.6 (C), 146.6 (CH), 150.2 (CH), 151.2 (C); LRMS (ESI+) *m*/*z* (%): 513 (100) [*M*]⁺.

### Procédure générale de synthèse de 3-(3-(4-azidobutoxy)propyl)-1-(4-(3-(pyridin-3-yl)propoxy)butyl)pyridinium chloride (17)

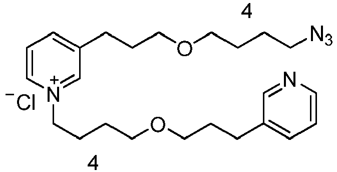

C₂₄H₃₆ClN₅O₂
MM = 461.5

Au sel de Zincke **14a** (112 mg, 0.26 mmol) dans le n-butanol est ajoutée l'amine **13a** (70 mg, 3.1 mmol). Le mélange est mis au reflux pendant 18 h. Après évaporation, le produit est purifié par flash chromatographie (7% to 10% méthanol/CH₂Cl₂, gel de silice) pour donner **17** (91 mg, 77%) sous la forme d'une huile brune.

R*_{f}* = 0.10 (10% MeOH/CH₂Cl₂); ¹H NMR (400 MHz, CDCl₃) *δ* 1.58-1.7 (m, 6H), 1.89 (m, 2H), 1.99 (m, 2H), 2.09 (m, 2H), 2.73 (m, 2H), 2.97 (m, 2H), 3.30 (m, 2H), 3.41-3.51 (m, 8H), 4.66 (t, *J* = 7.5 Hz, 2H), 7.37 (dd, *J* = 7.7, 4.8 Hz, 1H), 7.72 (d, *J* = 7.7 Hz, 1H), 8.03 (dd, *J* = 7.9, 6.2 Hz, 1H), 8.37 (m, 2H), 8.47 (d, *J* = 8.1 Hz, 1H), 8.87 (d, *J* = 6.2 Hz, 1H), 8.96 (s, 1H); ¹³C NMR (100 MHz, CDCl₃) *δ* 27.0 (CH₂), 27.5 (CH₂), 28.1 (CH₂), 30.0 (CH₂), 30.5 (CH₂), 30.8 (CH₂), 31.4 (CH₂), 32.2 (CH₂), 52.4 (CH₂), 62.9 (CH₂), 70.6 (CH₂), 70.9 (CH₂), 71.1 (CH₂), 71.4 (CH₂), 125.4 (CH), 129.1 (CH), 138.6 (CH), 139.8 (C), 143.6 (CH), 145.5 (C, CH), 146.9 (CH), 147.7 (CH), 150.2 (CH); LRMS (ESI+) *m*/*z* (%): 426 (100) [*M*⁺] ; 398 (30).

### 3-(3-(4-azidobutoxy)propyl)-1-(6-(3-(pyridin-3-yl)propoxy)hexyl)pyridinium chloride (18)

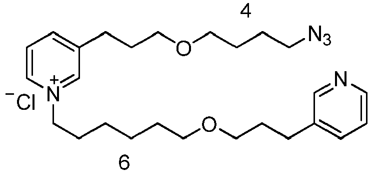

C₂₆H₄₀ClN₅O₂
MM = 489.5

A partir de **14a** (165 mg, 0.38 mmol) et de l'amine **13b** (115 mg, 0.49 mmol), le produit **18** (138 mg, 74%) est obtenu sous la forme d'une huile brune.

R*_{f}* = 0.17 (10% MeOH/CH₂Cl₂); ¹H NMR (400 MHz, CDCl₃) *δ* 1.38-1.49 (m, 6H), 1.54-1.74 (m, 4H), 1.86 (m, 2H), 1.95-2.09 (m, 4H), 2.67 (m, 2H), 2.93 (m, 2H), 3.30 (m, 2H), 3.38-3.45 (m, 6H), 4.67 (t, *J* = 7.6 Hz, 2H), 7.37 (dd, *J* = 7.7, 5.0 Hz, 1H), 7.71 (d, *J* = 7.7 Hz, 1H), 8.05 (dd, *J* = 7.8, 6.2 Hz, 1H), 8.34-8.41 (m, 2H), 8.49 (d, *J* = 8.1 Hz, 1H), 8.93 (d, *J* = 6.2 Hz, 1H), 9.02 (s, 1H); ¹³C NMR (100 MHz, CDCl₃) *δ* 26.9 (CH₂), 27.0 (CH₂), 27.1 (CH₂), 28.1 (CH₂), 30.4 (CH₂), 30.6 (CH₂), 30.7 (CH₂), 31.4 (CH₂), 32.2 (CH₂), 32.6 (CH₂), 52.4 (CH₂), 62.9 (CH₂), 70.6 (2×CH₂), 71.3 (CH₂), 71.7 (CH₂), 125.3 (CH), 129.0 (CH), 138.5 (CH), 139.7 (C), 143.5 (CH), 145.4 (CH), 145.5 (C), 146.8 (CH), 147.6 (CH), 150.2 (CH); LRMS (ESI+) *m*/*z* (%): 454 (100) [*M*⁺], 426 (45) .

### 3-(3-(6-azidohexyloxy)propyl)-1-(4-(3-(pyridin-3-yl)propoxy)butyl)pyridinium chloride (19)

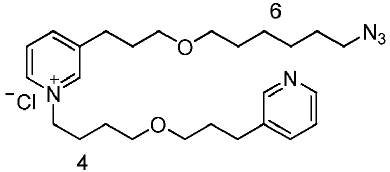

C₂₆H₄₀ClN₅O₂
MM = 489.5

A partir de **13b** (163 mg, 0.35 mmol) et de l'amine **12a** (92 mg, 0.42 mmol), le produit **19** (118 mg, 69%) est obtenu sous la forme d'une huile brune.

R*_{f}* = 0.12 (10% MeOH/CH₂Cl₂); ¹H NMR (400 MHz, CDCl₃) *δ* 1.30-1.43 (m, 4H), 1.49-1.61 (m, 4H), 1.66 (m, 2H), 1.89 (m, 2H), 2.00 (m, 2H), 2.12 (m, 2H), 2.73 (t, *J* = 7.5 Hz, 2H), 2.98 (t, *J* = 7.5 Hz, 2H), 3.27 (t, *J* = 7.0 Hz, 2H), 3.37-3.53 (m, 8H), 4.70 (m, 2H), 7.39 (m, 1H), 7.72 (d, *J* = 7.7 Hz, 1H), 8.05 (dd, *J =* 7.8, 6.2 Hz, 1H), 8.33-8.41 (m, 2H), 8.49 (d, *J* = 8.1 Hz, 1H), 8.92 (d, *J* = 6.2 Hz, 1H), 9.01 (s, 1H); ¹³C NMR (100 MHz, CDCl₃) *δ* 27.0 (CH₂), 27.4 (CH₂), 27.7 (CH₂), 30.0 (2×CH₂), 30.5 (CH₂), 30.7 (CH₂), 30.8 (CH₂), 31.4 (CH₂), 32.2 (CH₂), 52.5 (CH₂), 62.9 (CH₂), 70.6 (CH₂), 70.9 (CH₂), 71.1 (CH₂), 71.9 (CH₂), 125.4 (CH), 129.1 (CH), 138.4 (CH), 139.8 (C), 143.5 (CH), 145.4 (CH), 145.5 (C), 146.9 (CH), 147.6 (CH), 150.1 (CH); LRMS (ESI+) *m*/*z* (%) : 454 (100) [*M*⁺], 426 (35).

### 3-(3-(4-azidobutoxy)propyl)-1-(8-(3-(pyridin-3-yl)propoxy)octyl)pyridinium chloride (20)

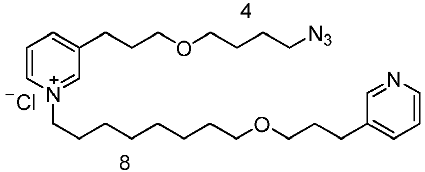

C₂₈H₄₄ClN₅O₂
MM= 517,5

A partir de **13a** (105 mg, 0.24 mmol) et de l'amine **14c** (79.5 mg, 0.3 mmol), le produit **20** (120 mg, 96%) est obtenu sous la forme d'une huile brune.

R*_{f}* = 0.13 (10% MeOH/CH₂Cl₂); ¹H NMR (400 MHz, CDCl₃) *δ* 1.30-1.46 (m, 8H), 1.51-1.71 (m, 4H), 1.87 (m, 2H), 1.95-2.06 (m, 4H), 2.73 (m, 2H), 2.97 (m, 2H), 3.30 (m, 2H), 3.37-3.46 (m, 6H), 3.49 (t, *J* = 6.0 Hz, 2H), 4.63 (m, 2H), 7.36 (dd, *J* = 7.7, 5.0 Hz, 1H), 7.71 (d, *J* = 7.7 Hz, 1H), 8.02 (dd, *J* = 7.8, 6.2 Hz, 1H), 8.34-8.39 (m, 2H), 8.47 (d, *J* = 8.1 Hz, 1H), 8.88 (d, *J* = 6.2 Hz, 1H), 8.97 (s, 1H); ¹³C NMR (100 MHz, CDCl₃) *δ* 27.0 (CH₂), 27.3 (CH₂), 27.6 (CH₂), 28.1 (CH₂), 28.7 (CH₂), 30.2 (CH₂), 30.3 (CH₂), 30.5 (2×CH₂), 30.8 (CH₂), 30.9 (CH₂), 32.2 (CH₂), 52.5 (CH₂), 63.1 (CH₂), 70.6 (2×CH₂), 71.5 (CH₂), 72.1 (CH₂), 125.4 (CH), 129.1 (CH), 138.6 (CH), 139.9 (C), 143.5 (CH), 145.5 (CH, C), 146.9 (CH), 147.6 (CH), 150.2 (CH); LRMS (ESI+) *m*/*z* (%): 482 (100) [*M*⁺], 454 (42).

### 3-(3-(6-azidohexyloxy)propyl)-1-(6-(3-(pyridin-3-yl)propoxy)hexyl)pyridinium chloride (21)

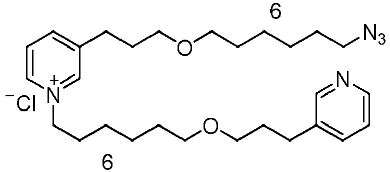

C₂₈H₄₄ClN₅O₂
MM = 517,5

A partir de **14b** (169 mg, 0.36 mmol) et de l'amine **13b** (103 mg, 0.44 mmol), le produit **21** (156 mg, 83%) est obtenu sous la forme d'une huile brune. R*_{f}* = 0.22 (15% MeOH/CH₂Cl₂); ¹H NMR (400 MHz, CDCl₃) *δ* 1.30-1.48 (m, 8H), 1.49-1.72 (m, 6H), 1.86 (m, 2H), 1.94-2.07 (m, 4H), 2.72 (t, *J* = 7.6 Hz, 2H), 2.97 (t, *J* = 7.6 Hz, 2H), 3.27 (m, 2H), 3.37-3.44 (m, 6H), 3.48 (t, *J* = 6.0 Hz, 2H), 4.63 (t, *J* = 7.5 Hz, 2H), 7.37 (dd, *J* = 7.6, 5.0 Hz, 1H), 7.71 (d, *J =* 7.6 Hz, 1H), 8.02 (m, 1H), 8.32-8.39 (m, 2H), 8.47 (d, *J* = 8.0 Hz, 1H), 8.88 (d, *J* = 6.0 Hz, 1H), 8.97 (s, 1H); ¹³C NMR (100 MHz, CDCl₃) *δ* 26.9 (CH₂), 27.0 (CH₂), 27.1 (CH₂), 27.8 (CH₂), 30.0 (CH₂), 30.5 (CH₂), 30.6 (CH₂), 30.7 (CH₂), 30.8 (CH₂), 31.4 (CH₂), 32.2 (CH₂), 32.7 (CH₂), 52.5 (CH₂), 63.0 (CH₂), 70.6 (CH₂), 70.7 (CH₂), 71.8 (CH₂), 71.9 (CH₂), 125.3 (CH), 129.1 (CH), 138.5 (CH), 139.8 (C), 143.5 (CH), 145.5 (CH, C), 146.9 (CH), 147.7 (CH), 150.3 (CH); LRMS (ESI+) *m*/*z* (%): 482 (100) [*M*⁺], 454 (60).

### 3-(3-(6-azidohexyloxy)propyl)-1-(8-(3-(pyridin-3-yl)propoxy)octyl)pyridinium chloride (22)

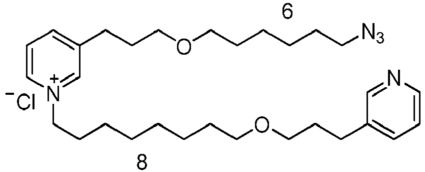

C₃₀H₄₈ClN₅O₂
MM = 545.5

A partir de **14b** (145 mg, 0.31 mmol) et de l'amine **13c** (99 mg, 0.38 mmol), le produit **22** (112 mg, 66%) est obtenu sous la forme d'une huile brune.

Rf = 0.11 (10% MeOH/CH₂Cl₂); ¹H NMR (400 MHz, CDCl₃) *δ* 1.31-1.45 (m, 12H), 1.51-1.62 (m, 6H), 1.87 (m, 2H), 1.94-2.06 (m, 4H), 2.73 (t, *J* = 7.6 Hz, 2H), 2.96 (t, *J* = 7.6 Hz, 2H), 3.28 (m, 2H), 3.38-3.44 (m, 6H), 3.48 (t, *J =* 6.0 Hz, 2H), 4.60 (t, *J* = 7.5 Hz, 2H), 7.36 (dd, *J* = 7.6, 5.0 Hz, 1H), 7.71 (d, *J* = 7.6 Hz, 1H), 8.01 (dd, *J* = 7.6, 6.0 Hz, 1H), 8.33-8.40 (m, 2H), 8.46 (d, *J* = 8.0 Hz, 1H), 8.84 (d, *J* = 6.0 Hz, 1H), 8.93 (s, 1H); ¹³C NMR (100 MHz, CDCl₃) *δ* 27.0 (CH₂), 27.2 (CH₂), 27.3 (CH₂), 27.8 (CH₂), 30.0 (CH₂), 30.2 (CH₂), 30.4 (CH₂), 30.5 (CH₂), 30.7 (2×CH₂), 30.8 (CH₂), 31.4 (CH₂), 32.2 (CH₂), 32.7 (CH₂), 52.5 (CH₂), 63.0 (CH₂), 70.6 (2×CH₂), 71.8 (CH₂), 71.9 (CH₂), 125.3 (CH), 129.1 (CH), 138.5 (CH), 139.8 (C), 143.5 (CH), 145.4 (CH, C), 146.8 (CH), 147.7 (CH), 150.2 (CH); LRMS (ESI+) *m*/*z* (%) : 510 (100) [*M*⁺] ; 482 (30) ; HRMS (ESI+) : calcd for C₃₀H₄₈N₅O₂ [*M*⁺] : 510.3802, found: 510.3802.

### 3-(3-(8-azidooctyloxy)propyl)-1-(6-(3-(pyridin-3-yl)propoxy)hexyl)pyridinium chloride (23)

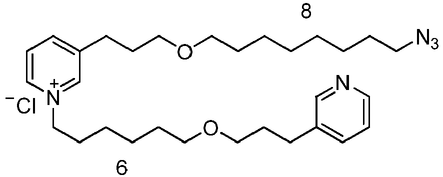

C₃₀H₄₈ClN₅O₂
MM = 545.5

A partir de **14c** (150 mg, 0.3 mmol) et de l'amine **13b** (86 mg, 0.36 mmol), le produit **23** (105 mg, 63%) est obtenu sous la forme d'une huile brune.

R*_{f}* = 0.13 (10% MeOH/CH₂Cl₂); ¹H NMR (400 MHz, CDCl₃) *δ* 1.29-1.48 (m, 12H), 1.50-1.63 (m, 6H), 1.87 (m, 2H), 1.93-2.07 (m, 4H), 2.72 (t, *J* = 7.6 Hz, 2H), 2.96 (t, *J* = 7.6 Hz, 2H), 3.26 (t, *J* = 7.0 Hz, 2H), 3.38-3.45 (m, 6H), 3.48 (t, *J* = 6.0 Hz, 2H), 4.61 (t, *J* = 7.5 Hz, 2H), 7.36 (dd, *J* = 7.6, 5.0 Hz, 1H), 7.71 (d, *J* = 7.6 Hz, 1H), 8.01 (dd, *J* = 7.8, 6.0 Hz, 1H), 8.34-8.39 (m, 2H), 8.46 (d, *J* = 8.0 Hz, 1H), 8.86 (d, *J* = 6.0 Hz, 1H), 8.94 (s, 1H) ; ¹³C NMR (100 MHz, CDCl₃) *δ* 26.9 (CH₂), 27.2 (CH₂), 27.3 (CH₂), 27.9 (CH₂), 30.0 (CH₂), 30.4 (CH₂), 30.5 (CH₂), 30.6 (CH₂), 30.7 (CH₂), 30.8 (CH₂), 30.9 (CH₂), 31.4 (CH₂), 32.2 (CH₂), 32.7 (CH₂), 52.6 (CH₂), 63.0 (CH₂), 70.6 (CH₂), 70.7 (CH₂), 71.8 (CH₂), 72.1 (CH₂), 125.3 (CH), 129.1 (CH), 138.5 (CH), 139.8 (C), 143.5 (CH), 145.6 (CH, C), 146.9 (CH), 147.7 (CH), 150.3 (CH); LRMS (ESI+) *m*/*z* (%): 510 (100) [*M*⁺]; 482 (30) .

### 3-(3-(10-azidodecyloxy)propyl)-1-(6-(3-(pyridin-3-yl)propoxy)hexyl)pyridinium chloride (24)

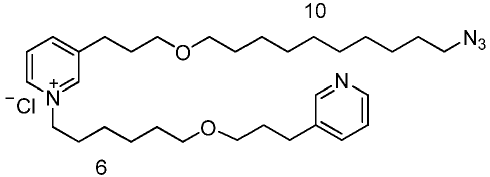

C₃₂H₅₂ClN₅O₂
MM = 573.4

A partir de **14d** (169 mg, 0.32 mmol) et de l'amine **13b** (92 mg, 0.39 mmol), le produit **24** (123 mg, 66%) est obtenu sous la forme d'une huile brune.

R*_{f}* = 0.16 (10% MeOH/CH₂Cl₂); ¹H NMR (400 MHz, CDCl₃) *δ* 1.27-1.47 (m, 16H), 1.48-1.62 (m, 6H), 1.86 (m, 2H), 1.95-2.08 (m, 4H), 2.72 (t, *J =* 7.6 Hz, 2H), 2.97 (t, *J =* 7.6 Hz, 2H), 3.26 (t, *J* = 7.0 Hz, 2H), 3.37-3.45 (m, 6H), 3.48 (t, *J* = 6.0 Hz, 2H), 4.65 (t, *J* = 7.5 Hz, 2H), 7.37 (dd, *J* = 7.6, 5.0 Hz, 1H), 7.71 (d, *J* = 7.6 Hz, 1H), 8.03 (dd, *J* = 7.8, 6.0 Hz, 1H), 8.33-8.41 (m, 2H), 8.47 (d, *J* = 8.0 Hz, 1H), 8.90 (d, *J* = 6.0 Hz, 1H), 8.99 (s, 1H); ¹³C NMR (100 MHz, CDCl₃) *δ* 26.9 (CH₂), 27.1 (CH₂), 27.4 (CH₂), 27.9 (CH₂), 30.0 (CH₂), 30.3 (CH₂), 30.5 (CH₂), 30.6 (CH₂), 30.7 (4×CH₂), 30.9 (CH₂), 31.4 (CH₂), 32.2 (CH₂), 32.6 (CH₂), 52.5 (CH₂), 63.0 (CH₂), 70.5 (CH₂), 70.6 (CH₂), 71.7 (CH₂), 72.1 (CH₂), 125.3 (CH), 129.0 (CH), 138.4 (CH), 139.7 (C), 143.5 (CH), 145.4 (CH, C), 146.8 (CH), 147.7 (CH), 150.2 (CH); HRMS (ESI+) *m*/*z* (%) : calcd for C₃₂H₅₂N₅O₂ [*M*⁺] : 538.4115, found: 538.4114.

### 3-(3-(8-azidooctyloxy)propyl)-1-(8-(3-(pyridin-3-yl)propoxy)octyl)pyridinium chloride (25)

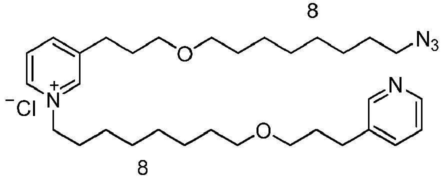

C₃₂H₅₂ClN₅O₂
MM = 573.5

A partir de **14c** (247 mg, 0.5 mmol) et de l'amine **13c** (165 mg, 0.63 mmol), produit **25** (262 mg, 91%) est obtenu sous la forme d'une huile brune.

R*_{f}* = 0.19 (10% MeOH/CH₂Cl₂); ¹H NMR (400 MHz, CDCl₃) *δ* 1.25-1.43 (m, 16H), 1.48-1.61 (m, 6H), 1.87 (m, 2H), 1.92-2.06 (m, 4H), 2.73 (t, *J* = 7.6 Hz, 2H), 2.96 (t, *J* = 7.6 Hz, 2H), 3.26 (t, *J* = 7.0 Hz, 2H), 3.37-3.44 (m, 6H), 3.48 (t, *J* = 6.0 Hz, 2H), 4.60 (t, *J* = 7.5 Hz, 2H), 7.36 (dd, *J* = 7.6, 5.0 Hz, 1H), 7.71 (d, *J* = 7.6 Hz, 1H), 8.01 (dd, *J* = 7.8, 6.0 Hz, 1H), 8.34-8.40 (m, 2H), 8.46 (d, *J* = 8.0 Hz, 1H), 8.85 (d, *J* = 6.0 Hz, 1H), 8.94 (s, 1H); ¹³C NMR (100 MHz, CDCl₃) *δ* 27.3 (3×CH₂), 27.9 (CH₂), 30.0 (CH₂), 30.2 (CH₂), 30.3 (CH₂), 30.4 (CH₂), 30.5 (CH₂), 30.6 (CH₂), 30.8 (CH₂), 30.9 (2×CH₂), 31.4 (CH₂), 32.2 (CH₂), 32.7 (CH₂), 52.6 (CH₂), 63.1 (CH₂), 70.6 (2×CH₂), 72.0 (CH₂), 72.1 (CH₂), 125.3 (CH), 129.1 (CH), 138.6 (CH), 139.8 (C), 143.5 (CH), 145.5 (CH, C), 146.9 (CH), 147.7 (CH), 150.3 (CH); LRMS (ESI+) *m*/*z* (%): 538 (100) [*M*⁺] ; 510 (18).

### 3-(3-(12-azidododecyloxy)propyl)-1-(4-(3-(pyridin-3-yl)propoxy)butyl)pyridinium chloride (26)

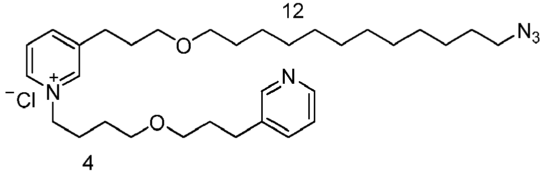

C₃₂H₅₂ClN₅O₂
MM = 573.5

A partir de **14e** (278 mg, 0.51 mmol) et de l'amine **13a** (133 mg, 0.61 mmol), le produit **26** (197 mg, 68%) est obtenu sous la forme d'une huile brune.

R*_{f}* = 0.22 (10% MeOH/CH₂Cl₂); ¹H NMR (400 MHz, CDCl₃) *δ* 1.24-1.40 (m, 16H), 1.47-1.60 (m, 4H), 1.66 (m, 2H), 1.89 (m, 2H), 2.00 (m, 2H), 2.12 (m, 2H), 2.74 (t, *J* = 7.6 Hz, 2H), 2.98 (t, *J* = 7.6 Hz, 2H), 3.26 (t, *J* = 7.0 Hz, 2H), 3.40 (t, *J* = 6.4 Hz, 2H), 3.43-3.53 (m, 6H), 4.71 (t, *J* = 7.5 Hz, 2H), 7.40 (dd, *J* = 7.6, 5.0 Hz, 1H), 7.75 (d, *J* = 7.6 Hz, 1H), 8.06 (dd, *J* = 7.8, 6.0 Hz, 1H), 8.34-8.44 (m, 2H), 8.49 (d, *J* = 8.0 Hz, 1H), 8.94 (d, *J* = 6.0 Hz, 1H), 9.03 (s, 1H); ¹³C NMR (100 MHz, CDCl₃) *δ* 27.4 (3×CH₂), 27.9 (CH₂), 29.9 (CH₂), 30.0 (CH₂), 30.4 (CH₂), 30.5 (CH₂), 30.7 (3×CH₂), 30.8 (2×CH₂), 30.9 (CH₂), 31.4 (CH₂), 32.2 (CH₂), 52.5 (CH₂), 62.8 (CH₂), 70.5 (CH₂), 70.8 (CH₂), 71.0 (CH₂), 72.1 (CH₂), 125.4 (CH), 129.1 (CH), 138.8 (CH), 139.9 (C), 143.5 (CH), 145.4 (CH), 145.5 (C), 146.9 (CH), 147.4 (CH), 149.9 (CH); HRMS (ESI+) *m*/*z* (%) : calcd for C₃₂H₅₂N₅O₂ [*M*⁺] : 538.4115, found: 538.4113.

### 3-(3-(6-azidohexyloxy)propyl)-1-(10-(3-(pyridin-3-yl)propoxy)decyl)pyridinium chloride (27)

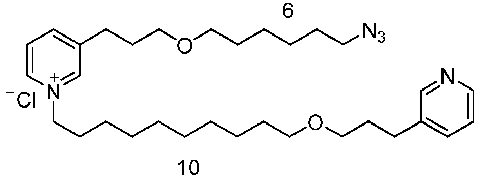

C₃₂H₅₂ClN₅O₂
MM = 573.5

A partir de **14b** (117.6 mg, 0.25 mmol) et de l'amine **13d** (89 mg, 0.3 mmol), le produit **27** (212 mg, 64%) est obtenu sous la forme d'une huile brune. Rf = 0.20 (10% MeOH/CH₂Cl₂); ¹H NMR (400 MHz, CDCl₃) *δ* 1.26-1.43 (m, 16H), 1.50-1.62 (m, 6H), 1.87 (m, 2H), 1.96-2.07 (m, 4H), 2.73 (t, *J* = 7.6 Hz, 2H), 2.98 (t, *J* = 7.6 Hz, 2H), 3.28 (t, *J* = 7.0 Hz, 2H), 3.37-3.44 (m, 6H), 3.48 (t, *J* = 6.0 Hz, 2H), 4.65 (t, *J* = 7.5 Hz, 2H), 7.36 (dd, *J* = 7.6, 5.0 Hz, 1H), 7.70 (d, *J* = 7.6 Hz, 1H), 8.05 (dd, *J =* 7.8, 6.0 Hz, 1H), 8.33-8.41 (m, 2H), 8.48 (d, *J* = 8.0 Hz, 1H), 8.92 (d, *J* = 6.0 Hz, 1H), 9.01 (s, 1H); ¹³C NMR (100 MHz, CDCl₃) *δ* 27.0 (CH₂), 27.3 (CH₂), 27.4 (CH₂), 27.8 (CH₂), 30.0 (CH₂), 30.2 (CH₂), 30.5 (CH₂), 30.6 (CH₂), 30.7 (2×CH₂), 30.8 (2×CH₂), 30.9 (CH₂), 31.4 (CH₂), 32.2 (CH₂), 32.7 (CH₂), 52.5 (CH₂), 63.0 (CH₂), 70.5 (CH₂), 70.6 (CH₂), 71.8 (CH₂), 72.0 (CH₂), 125.2 (CH), 129.0 (CH), 138.3 (CH), 139.7 (C), 143.5 (CH), 145.4 (CH), 145.5 (C), 146.8 (CH), 147.7 (CH), 150.3 (CH); HRMS (ESI+) *m*/*z* (%) : calcd for C₃₂H₅₂N₅O₂ [*M*⁺] : 538.4115, found: 538.4114.

### 3-(3-(6-azidohexyloxy)propyl)-1-(12-(3-(pyridin-3-yl)propoxy)dodecyl)pyridinium chloride (28)

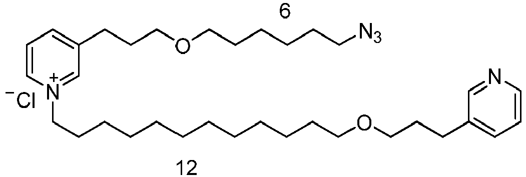

C₃₄H₅₆ClN₅O₂
MM = 601.5

A partir de **14b** (95 mg, 0.2 mmol) et de l'amine **13e** (82 mg, 0.25 mmol), le produit **28** (104 mg, 84%) est obtenu sous la forme d'une huile brune. R*_{f}* = 0.19 (10% MeOH/CH₂Cl₂); ¹H NMR (400 MHz, CDCl₃) *δ* 1.29-1.42 (m, 20H), 1.50-1.63 (m, 6H), 1.88 (m, 2H), 1.94-2.05 (m, 4H), 2.73 (t, *J* = 7.6 Hz, 2H), 2.96 (t, *J* = 7.6 Hz, 2H), 3.28 (t, *J* = 7.0 Hz, 2H), 3.38-3.44 (m, 6H), 3.48 (t, *J* = 6.0 Hz, 2H), 4.58 (t, *J* = 7.5 Hz, 2H), 7.36 (dd, *J* = 7.6, 5.0 Hz, 1H), 7.71 (d, *J* = 7.6 Hz, 1H), 8.00 (dd, *J =* 7.8, 6.0 Hz, 1H), 8.33-8.41 (m, 2H), 8.46 (d, *J* = 8.0 Hz, 1H), 8.83 (d, *J* = 6.0 Hz, 1H), 8.91 (s, 1H); ¹³C NMR (100 MHz, CDCl₃) *δ* 27.0 (CH₂), 27.3 (CH₂), 27.5 (CH₂), 27.8 (CH₂), 30.0 (CH₂), 30.3 (CH₂), 30.5 (CH₂), 30.6 (CH₂), 30.7 (3×CH₂), 30.8 (3×CH₂), 30.9 (CH₂), 31.4 (CH₂), 32.2 (CH₂), 32.7 (CH₂), 52.5 (CH₂), 63.1 (CH₂), 70.6 (CH₂), 70.7 (CH₂), 72.0 (CH₂), 72.1 (CH₂), 125.3 (CH), 129.1 (CH), 138.5 (CH), 139.8 (C), 143.4 (CH), 145.5 (C), 145.6 (CH), 146.9 (CH), 147.7 (CH), 150.3 (CH); HRMS (ESI+) *m*/*z* (%): calcd for C₃₄H₅₆N₅O₂ [*M*⁺]: 566.4428, found: 566.4425.

### 3-(3-(8-azidooctyloxy)propyl)-1-(10-(3-(pyridin-3-yl)propoxy)decyl)pyridinium chloride (29)

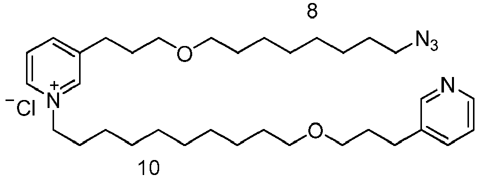

C₃₄H₅₆ClN₅O₂
MM = 601.5

A partir de **14c** (217 mg, 0.44 mmol) et de l'amine **13d** (155 mg, 0.53 mmol), le produit **29** (172 mg, 65%) est obtenu sous la forme d'une huile brune. R*_{f}* = 0.20 (10% MeOH/CH₂Cl₂); ¹H NMR (400 MHz, CDCl₃) *δ* 1.25-1.41 (m, 20H), 1.49-1.61 (m, 6H), 1.88 (m, 2H), 1.95-2.05 (m, 4H), 2.73 (t, *J* = 7.6 Hz, 2H), 2.97 (t, *J* = 7.6 Hz, 2H), 3.27 (t, *J* = 7.0 Hz, 2H), 3.37-3.45 (m, 6H), 3.48 (t, *J* = 6.0 Hz, 2H), 4.62 (t, *J* = 7.5 Hz, 2H), 7.36 (dd, *J* = 7.6, 5.0 Hz, 1H), 7.71 (d, *J* = 7.6 Hz, 1H), 8.02 (dd, *J* = 7.8, 6.0 Hz, 1H), 8.32-8.40 (m, 2H), 8.47 (d, *J* = 8.0 Hz, 1H), 8.87 (d, *J* = 6.0 Hz, 1H), 8.96 (s, 1H); ¹³C NMR (100 MHz, CDCl₃) *δ* 27.3 (2×CH₂), 27.5 (CH₂), 27.9 (CH₂), 30.0 (CH₂), 30.3 (CH₂), 30.4 (CH₂), 30.5 (CH₂), 30.6 (2×CH₂), 30.7 (2×CH₂), 30.8 (CH₂), 30.9 (2×CH₂), 31.4 (CH₂), 32.3 (CH₂), 32.7 (CH₂), 52.6 (CH₂), 63.1 (CH₂), 70.6 (2×CH₂), 72.1 (2×CH₂), 125.3 (CH), 129.1 (CH), 138.5 (CH), 139.8 (C), 143.5 (CH), 145.5 (CH, C), 146.9 (CH), 147.7 (CH), 150.3 (CH); LRMS (ESI+) *m*/*z* (%): 566 (100) [*M*⁺] ; 538 (24) .

### 3-(3-(10-azidodecyloxy)propyl)-1-(10-(3-(pyridin-3-yl)propoxy)decyl)pyridinium chloride (30)

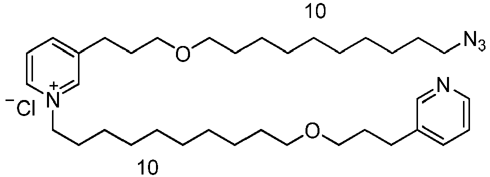

C₃₆H₆₀ClN₅O₂
MM = 629.5

A partir de **14d** (284 mg, 0.55 mmol) et de l'amine **13d** (208 mg, 0.71 mmol), le produit **30** (199 mg, 91%) est obtenu sous la forme d'une huile brune. Rf = 0.12 (10% MeOH/CH₂Cl₂); ¹H NMR (400 MHz, CDCl₃) *δ* 1.26-1.41 (m, 24H), 1.48-1.62 (m, 6H), 1.87 (m, 2H), 1.95-2.06 (m, 4H), 2.73 (t, *J* = 7.6 Hz, 2H), 2.97 (t, *J* = 7.6 Hz, 2H), 3.26 (t, *J* = 7.0 Hz, 2H), 3.37-3.44 (m, 6H), 3.48 (t, *J* = 6.0 Hz, 2H), 4.63 (t, *J* = 7.5 Hz, 2H), 7.36 (dd, *J* = 7.6, 5.0 Hz, 1H), 7.71 (d, *J* = 7.6 Hz, 1H), 8.03 (dd, *J* = 7.8, 6.0 Hz, 1H), 8.34-8.39 (m, 2H), 8.47 (d, *J* = 8.0 Hz, 1H), 8.89 (d, *J* = 6.0 Hz, 1H), 8.97 (s, 1H); ¹³C NMR (100 MHz, CDCl₃) *δ* 27.3 (CH₂), 27.4 (CH₂), 27.5 (CH₂), 27.9 (CH₂), 30.0 (CH₂), 30.3 (CH₂), 30.4 (CH₂), 30.5 (CH₂), 30.6 (CH₂), 30.7 (5×CH₂), 30.8 (CH₂), 30.9 (2×CH₂), 31.4 (CH₂), 32.3 (CH₂), 32.7 (CH₂), 52.6 (CH₂), 63.0 (CH₂), 70.6 (2×CH₂), 72.1 (2×CH₂), 125.3 (CH), 129.0 (CH), 138.4 (CH), 139.7 (C), 143.5 (CH), 145.4 (CH), 145.5 (C), 146.8 (CH), 147.7 (CH), 150.3 (CH); LRMS (ESI+) *m*/*z* (%) : 594 (100) [*M*]⁺, 566 (6). HRMS (ESI+) *m*/*z* (%) : calcd for C₃₆H₆₀N₅O₂ [*M*⁺] : 594.4741, found: 594.4737.

### 3-(3-(12-azidododecyloxy)propyl)-1-(8-(3-(pyridin-3-yl)propoxy)octyl)pyridinium chloride (31)

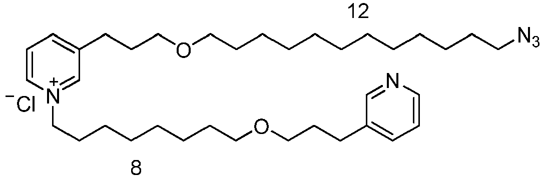

C₃₆H₆₀ClN₅O₂
MM = 629.5

A partir de **14e** (311 mg, 0.57 mmol) et de l'amine **13c** (187 mg, 0.71 mmol), le produit **31** (344 mg, 96%) est obtenu sous la forme d'une huile brune. R*_{f}* = 0.28 (10% MeOH/CH₂Cl₂); ¹H NMR (400 MHz, CDCl₃) *δ* 1.26-1.43 (m, 24H), 1.49-1.60 (m, 6H), 1.87 (m, 2H), 1.95-2.07 (m, 4H), 2.72 (t, *J* = 7.6 Hz, 2H), 2.97 (t, *J* = 7.6 Hz, 2H), 3.26 (t, *J* = 7.0 Hz, 2H), 3.37-3.44 (m, 6H), 3.48 (t, *J* = 6.0 Hz, 2H), 4.64 (t, *J* = 7.5 Hz, 2H), 7.36 (dd, *J* = 7.6, 5.0 Hz, 1H), 7.71 (d, *J* = 7.6 Hz, 1H), 8.03 (dd, *J =* 7.8, 6.0 Hz, 1H), 8.33-8.40 (m, 2H), 8.47 (d, *J* = 8.0 Hz, 1H), 8.89 (d, *J* = 6.0 Hz, 1H), 8.98 (s, 1H); ¹³C NMR (100 MHz, CDCl₃) *δ* 27.3 (2×CH₂), 27.4 (CH₂), 27.9 (CH₂), 30.0 (CH₂), 30.2 (CH₂), 30.4 (2×CH₂), 30.5 (CH₂), 30.7 (4×CH₂), 30.8 (CH₂), 30.9 (2×CH₂), 31.0 (CH₂), 31.4 (CH₂), 32.2 (CH₂), 32.7 (CH₂), 52.6 (CH₂), 63.0 (CH₂), 70.6 (2×CH₂), 72.0 (CH₂), 72.1 (CH₂), 125.3 (CH), 129.0 (CH), 138.4 (CH), 139.7 (C), 143.5 (CH), 145.4 (CH, C), 146.8 (CH), 147.4 (CH), 150.2 (CH); HRMS (ESI+) *m*/*z* (%) : calcd for C₃₀H₄₈N₅O₂ [*M*⁺] : 594.4741, found: 594.4739.

### 3-(3-(10-azidodecyloxy)propyl)-1-(12-(3-(pyridin-3-yl)propoxy)dodecyl)pyridinium chloride (32)

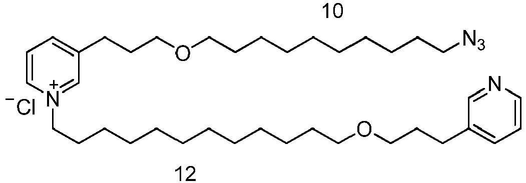

C₃₈H₆₄ClN₅O₂
MM = 657.5

A partir de **14d** (135 mg, 0.26 mmol) et de l'amine **13e** (104 mg, 0.32 mmol), le produit **32** (111 mg, 65%) est obtenu sous la forme d'une huile brune. R*_{f}* = 0.25 (10% MeOH/CH₂Cl₂); ¹H NMR (400 MHz, CDCl₃) δ 1.26-1.42 (m, 28H), 1.48-1.60 (m, 6H), 1.87 (m, 2H), 1.95-2.07 (m, 4H), 2.73 (t, *J* = 7.6 Hz, 2H), 2.97 (t, *J* = 7.6 Hz, 2H), 3.26 (t, *J* = 7.0 Hz, 2H), 3.37-3.44 (m, 6H), 3. 48 (t, *J* = 6.0 Hz, 2H), 4.64 (t, *J* = 7.5 Hz, 2H), 7.36 (dd, *J* = 7.6, 5.0 Hz, 1H), 7.70 (d, *J* = 7.6 Hz, 1H), 8.03 (dd, *J* = 7.8, 6.0 Hz, 1H), 8.33-8.40 (m, 2H), 8.47 (d, *J* = 8.0 Hz, 1H), 8.90 (d, *J* = 6.0 Hz, 1H), 8.98 (s, 1H); ¹³C NMR (100 MHz, CDCl₃) δ 27.3 (CH₂), 27.4 (2×CH₂), 27.9 (CH₂), 30.0 (CH₂), 30.2 (CH₂), 30.4 (CH₂), 30.5 (CH₂), 30.6 (CH₂), 30.7 (8×CH₂), 30.8 (CH₂), 30.9 (CH₂), 31.4 (CH₂), 32.2 (CH₂), 32.7 (CH₂), 52.5 (CH₂), 63.0 (CH₂), 70.5 (CH₂), 70.6 (CH₂), 72.0 (CH₂), 72.1 (CH₂), 125.3 (CH), 129.0 (CH), 138.4 (CH), 139.7 (C), 143.5 (CH), 145.4 (CH), 145.5 (C), 146.8 (CH), 147.7 (CH), 150.3 (CH); LRMS (ESI+) *m*/*z* (%) : 622 (100) [*M*⁺] ; 594 (12).

### 3-(3-(12-azidododecyloxy)propyl)-1-(10-(3-(pyridin-3-yl)propoxy)decyl)pyridinium chloride (33)

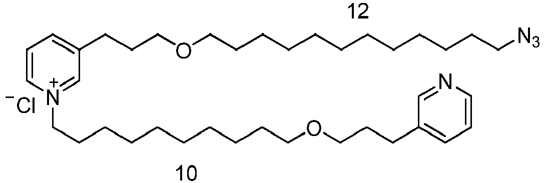

C₃₈H₆₄ClN₅O₂
MM = 657.5

A partir de **14e** (138 mg, 0.25 mmol) et de l'aminé **13d** (93 mg, 0.32 mmol), le produit **33** (93 mg, 56%) est obtenu sous la forme d'une huile brune. R*_{f}* = 0.14 (10% MeOH/CH₂Cl₂); ¹H NMR (400 MHz, CDCl₃) δ 1.24-1.41 (m, 28H), 1.48-1.60 (m, 6H), 1.87 (m, 2H), 1.94-2.04 (m, 4H), 2.73 (t, *J* = 7.6 Hz, 2H), 2.97 (t, *J* = 7.6 Hz, 2H), 3.26 (t, *J* = 7.0 Hz, 2H), 3.36-3.45 (m, 6H), 3.47 (t, *J* = 6.0 Hz, 2H), 4.61 (t, *J* = 7.5 Hz, 2H), 7.36 (dd, *J* = 7.6, 5.0 Hz, 1H), 7. 71 (d, *J* = 7.6 Hz, 1H), 8.02 (dd, *J* = 7.8, 6.0 Hz, 1H), 8.32-8.41 (m, 2H), 8.46 (d, *J* = 8.0 Hz, 1H), 8.86 (d, *J* = 6.0 Hz, 1H), 8.95 (s, 1H); ¹³C NMR (100 MHz, CDCl₃) δ 27.3 (CH₂), 27.5 (2×CH₂), 28.0 (CH₂), 30.1 (CH₂), 30.3 (CH₂), 30.4 (CH₂), 30.5 (CH₂), 30.6 (CH₂), 30.7 (4×CH₂), 30.8 (4×CH₂), 30.9 (CH₂), 31.0 (CH₂), 31.4 (CH₂), 32.3 (CH₂), 32.7 (CH₂), 52.6 (CH₂), 63.1 (CH₂), 70.6 (2×CH₂), 72.1 (CH₂), 72.2 (CH₂), 125.3 (CH), 129.1 (CH), 138.5 (CH), 139.8 (C), 143.5 (CH), 145.5 (CH, C), 146.9 (CH), 147.7 (CH), 150.2 (CH); LRMS (ESI+) *m*/*z* (%): 658 (100) [*M*⁺] ; 630 (10).

### 3-(3-(12-azidododecyloxy)propyl)-1-(12-(3-(pyridin-3-yl)propoxy)dodecyl)pyridinium chloride (34)

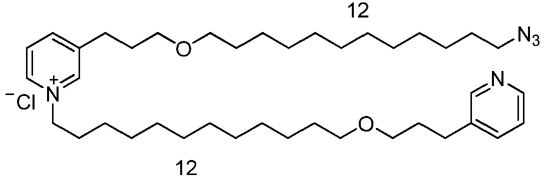

C₄₀H₆₈ClN₅O₂
MM = 685.5

A partir de **14e** (138 mg, 0.25 mmol) et de l'amine **13e** (105 mg, 0.33 mmol), le produit **34** (126 mg, 73%) est obtenu sous la forme d'une huile brune. R*_{f}* = 0.14 (10% MeOH/CH₂Cl₂); ¹H NMR (400 MHz, CDCl₃) *δ* 1.26-1.42 (m, 32H), 1.47-1.60 (m, 6H), 1.87 (m, 2H), 1.95-2.06 (m, 4H), 2.73 (t, *J* = 7.6 Hz, 2H), 2.97 (t, *J* = 7.6 Hz, 2H), 3.26 (t, *J* = 7.0 Hz, 2H), 3.36-3.44 (m, 6H), 3.48 (t, *J* = 6.0 Hz, 2H), 4.63 (t, *J* = 7.5 Hz, 2H), 7.36 (dd, *J* = 7.6, 5.0 Hz, 1H), 7.71 (d, *J* = 7.6 Hz, 1H), 8.03 (dd, *J* = 7.8, 6.0 Hz, 1H), 8.34-8.40 (m, 2H), 8.47 (d, *J* = 8.0 Hz, 1H), 8.89 (d, *J* = 6.0 Hz, 1H), 8.98 (s, 1H); ¹³C NMR (100 MHz, CDCl₃) *δ* 27.3 (CH₂), 27.4 (2×CH₂), 27.9 (CH₂) , 30.0 (CH₂) , 30.2 (CH₂), 30.4 (CH₂), 30.5 (CH₂), 30.6 (CH₂), 30.7 (3×CH₂), 30.8 (6×CH₂), 30.9 (2×CH₂), 31.0 (CH₂), 31.4 (CH₂), 32.2 (CH₂), 32.7 (CH₂), 52.5 (CH₂), 63.0 (CH₂), 70.5 (CH₂), 70.6 (CH₂), 72.0 (CH₂), 72.1 (CH₂), 125.3 (CH), 129.0 (CH), 138.4 (CH), 139.7 (C), 143.5 (CH), 145.4 (CH), 145.5 (C), 146.8 (CH), 147.7 (CH), 150.2 (CH) ; HRMS (ESI+) *m*/*z* (%): calcd for C₄₀H₆₈N₅O₂ [*M*⁻¹] : 650.5360, found: 650.5367.

Hu, M., Li, J., and Yao, S. Q. In Situ "Click" Assembly of Small Molecule Matrix Metalloprotease Inhibitors Containing Zinc-Chelating Groups, Org. Lett. 2008, 10, 5529-5531.

Fletcher, J. T., Walz, S. E., and Keeney, M. E. Monosubstituted 1,2,3-triazoles from two-step one-pot deprotection/click additions of trimethylsilylacetylene, Tetrahedron Lett. 2008, 49, 7030-7032.

Fletcher, J. T., Bumgarner, B. J., Engels, N. D., and Skoglund, D. A. Multidentate 1,2,3-Triazole-Containing Chelators from Tandem Deprotection/Click Reactions of (Trimethylsilyl)alkynes and Comparison of Their Ruthenium(II) Complexes, Organometallics 2008, 27, 5430-5433.

### Procédure générale de synthèse de 3-(3-(4-(4-(8-acetoxy-5-chloroquinolin-7-yl)-1H-1,2,3-triazol-1-yl)butoxy)propyl)-1-(4-(3-(pyridin-3-yl)propoxy)butyl)pyridinium chloride (35)

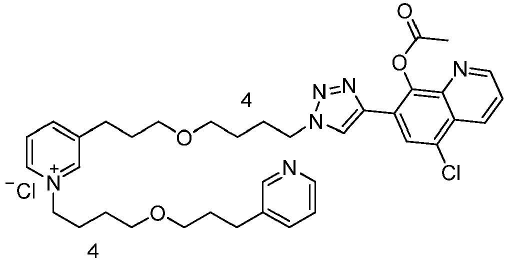

C₃₇H₄₄Cl₂N₆O₄
MM = 706

Au sel de Pyridine-pyridinium **17** (30.4 mg, 0.066 mmol) et l'acetylène TMS-protégé **8** (26 mg, 0.082 mmol) dans le tBuOH (0.4 mL)/H₂O (0.4 mL) est ajouté CuSO₄.5H₂O (3.3 mg, 0.013 mmol), sodium ascorbate (5.1 mg, 0.026 mmol) et K₂CO₃ (9.12 mg, 0.06 mmol). Le mélange est agité pendant 8 h à tempéraure ambiante. EtOAc est ajouté et la solution est filtrée. Après évaporation du solvant, le produit est purifié par flash chromatographie (7% to 10% methanol/CH₂Cl₂, gel de silice) pour donne **35** (19 mg, 41%) sous la forme d'une huile brune.

Rf = 0.11 (10% MeOH/CH₂Cl₂); ¹H NMR (400 MHz, CDCl₃) *δ* 1.30-1.48 (m, 2H), 1.57-1.70 (m, 2H), 1.85 (m, 2H), 1.95-2.15 (m, 6H), 2.60 (s, 3H), 2.66 (t, *J* = 7.6 Hz, 2H), 2.97 (m, 2H), 3.37-3.48 (m, 8H), 4.53 (t, *J* = 7.0 Hz, 2H), 4.97 (t, *J* = 7.2 Hz, 2H), 7.25 (m, 1H), 7.50-7.56 (m, 2H), 7.92 (dd, *J* = 7.8, 6.6 Hz, 1H), 8.14 (s, 1H), 8.21 (d, *J* = 7.8 Hz, 1H), 8.40-8.45 (m, 2H), 8.53 (s, 1H), 8.55 (dd, *J* = 8.0, 1.1 Hz, 1H), 8.95 (dd, *J* = 4.1, 1.1 Hz, 1H), 9.21 (d, *J* = 5.5 Hz, 1H), 9.49 (s, 1H) ; ¹³C NMR (100 MHz, CDCl₃) *δ* 21.6 (CH₃), 26.3 (CH₂), 26.6 (CH₂), 26.7 (CH₂), 27.7 (CH₂), 29.9 (CH₂), 30.2 (CH₂), 30.9 (CH₂), 32.1 (CH₂), 50.6 (CH₂), 61.8 (CH₂), 69.4 (CH₂), 70.0 (CH₂), 70.1 (CH₂), 70.2 (CH₂), 122.6 (CH), 123.5 (CH), 123.9 (C), 125.2 (CH), 125.5 (CH), 126.9 (C), 127.8 (CH), 129.3 (C), 133.3 (CH), 135.4 (C), 136.7 (CH), 141.6 (C), 142.1 (C), 142.6 (C, CH), 144.1 (C), 144.9 (CH), 145.0 (CH), 146.9 (CH), 149.6 (CH), 151.4 (CH), 169.4 (C); LRMS (ESI+) *m*/*z* (%): 671 (2) [*M*⁺] ; 629 (100).

### 3-(3-(4-(4-(8-acetoxy-5-chloroquinolin-7-yl)-1H-1,2,3-triazol-1-yl)butoxy)propyl)-1-(6-(3-(pyridin-3-yl)propoxy)hexyl)pyridinium chloride (36)

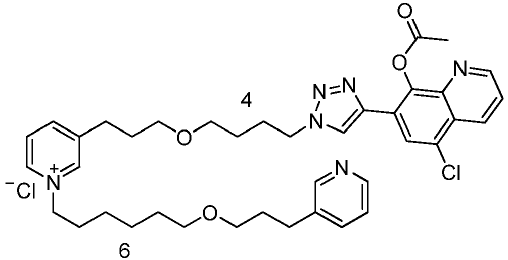

C₃₉H₄₈Cl₂N₆O₄
MM = 734

A partir de pyridine-pyridinium chloride **18** (91.5 mg, 0.18 mmol) et de l'acetylène TMS-protégé **8** (74 mg, 0.22 mmol), le produit **36** (65 mg, 47%) est obtenu sous la forme d'une huile brune. R*_{f}* = 0.11 (10% MeOH/CH₂Cl₂); ¹H NMR (400 MHz, CDCl₃) *δ* 1.30-1.37 (m, 4H), 1.50 (m, 2H), 1.60 (m, 2H), 1.85 (m, 2H), 1.95-2.08 (m, 6H), 2.60 (s, 3H), 2.67 (t, *J* = 7.6 Hz, 2H), 2.96 (t, *J* = 7.5 Hz, 2H), 3.30-3.37 (m, 4H), 3.40-3.47 (m, 4H), 4.54 (t, *J* = 7.0 Hz, 2H), 4.93 (t, *J* = 7.0 Hz, 2H), 7.23 (dd, *J* = 7.7, 4.8 Hz, 1H), 7.50-7.56 (m, 2H), 7.98 (t, J = 6.7 Hz, 1H), 8.21 (s, 1H), 8.23 (d, *J* = 8.1 Hz, 1H), 8.40-8.45 (m, 2H), 8.51 (s, 1H), 8.53 (dd, *J* = 8.8, 1.4 Hz, 1H), 8.94 (dd, *J* = 4.1, 1.4 Hz, 1H), 9.31 (d, *J* = 5.3 Hz, 1H), 9.48 (s, 1H) ; ¹³C NMR (100 MHz, CDCl₃) *δ* 21.5 (CH₃), 25.8 (CH₂), 26.0 (CH₂), 26.6 (CH₂), 27.6 (CH₂), 29.4 (CH₂), 29.5 (CH₂), 29.7 (CH₂), 30.1 (CH₂), 30.9 (CH₂), 32.1 (CH₂), 50.4 (CH₂), 61.7 (CH₂), 69.3 (CH₂), 69.4 (CH₂), 70.0 (CH₂), 70.6 (CH₂), 122.5 (CH), 123.5 (CH), 123.6 (CH), 123.8 (C), 125.3 (CH), 126.7 (C), 127.9 (CH), 129.1 (C), 133.1 (CH), 136.4 (CH), 137.5 (C), 141.5 (C), 142.0 (C), 142.5 (C, CH), 142.7 (C), 143.8 (C), 144.6 (CH), 144.9 (CH), 146.9 (CH), 149.6 (CH), 151.3 (CH), 169.3 (C); LRMS (ESI+) *m*/*z* (%) : 699 (2) [*M*⁺] ; 657 (100).

### 3-(3-(6-(4-(8-acetoxy-5-chloroquinolin-7-yl)-1H-1,2,3-triazol-1-yl)hexyloxy)propyl)-1-(4-(3-(pyridin-3-yl)propoxy)butyl)pyridinium chloride (37)

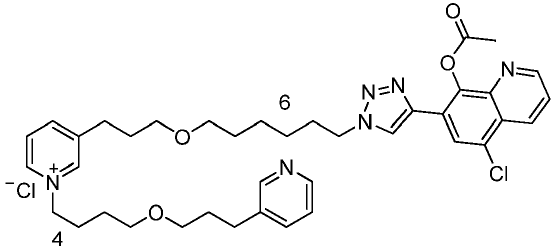

C₃₉H₄₈Cl₂N₆O₄
MM =734

A partir de pyridine-pyridinium chloride **19** (89 mg, 0.18 mmol) et de l'acetylène TMS-protégé **8** (74 mg, 0.22 mmol), le produit **37** (41 mg, 31%) est obtenu sous la forme d'une huile brune. R*_{f}* = 0.07 (10% MeOH/CH₂Cl₂); ¹H NMR (400 MHz, CDCl₃) *δ* 1.35-1.40 (m, 4H), 1.53 (m, 2H), 1.64 (m, 2H), 1.86 (m, 2H), 1.93-2.02 (m, 4H), 2.13 (m, 2H), 2.59 (s, 3H), 2.69 (t, *J* = 7.5 Hz, 2H), 2.96 (m, 2H), 3.35-3.47 (m, 8H), 4.49 (t, *J* = 7.0 Hz, 2H), 5.00 (t, *J* = 6.7 Hz, 2H), 7.32 (m, 1H), 7.51-7.60 (m, 2H), 7.97 (m, 1H), 8.08 (s, 1H), 8.21 (d, *J* = 7.8 Hz, 1H), 8.38-8.45 (m, 2H), 8.52 (s, 1H), 8.55 (dd, *J* = 8.5, 1.5 Hz, 1H), 8.95 (dd, *J* = 4.2, 1.5 Hz, 1H), 9.37-9.42 (m, 2H); ¹³C NMR (100 MHz, CDCl₃) *δ* 21.5 (CH₃), 25.8 (CH₂), 26.3 (CH₂), 26.4 (CH₂), 29.5 (4×CH₂), 29.9 (CH₂), 30.3 (CH₂), 30.4 (CH₂), 30.9 (CH₂), 50.7 (CH₂), 61.7 (CH₂), 69.3 (CH₂), 69.9 (CH₂), 70.0 (CH₂), 70.9 (CH₂), 122.6 (CH), 123.2 (CH), 123.9 (C, CH), 125.4 (CH), 126.8 (C), 127.9 (CH), 129.2 (C), 133.3 (CH), 136.9 (CH), 137.6 (C), 141.6 (C), 142.1 (C), 142.5 (C), 142.9 (CH), 144.1 (C), 144.7 (CH), 144.9 (CH), 146.4 (CH), 149.1 (CH), 151.4 (CH), 169.2 (C) ; LRMS (ESI+) *m*/*z* (%): 699 (2) [*M*⁺] ; 657 (100); HRMS (ESI+) *m*/*z* (%): calcd for C₃₉H₄₈ClN₆O₄ [*M*⁺] : 699.3420, found: 699.3423.

### 3-(3-(4-(4-(8-acetoxy-5-chloroquinolin-7-yl)-1H-1,2,3-triazol-1-yl)butoxy)propyl)-1-(8-(3-(pyridin-3-yl)propoxy)octyl)pyridinium chloride (38)

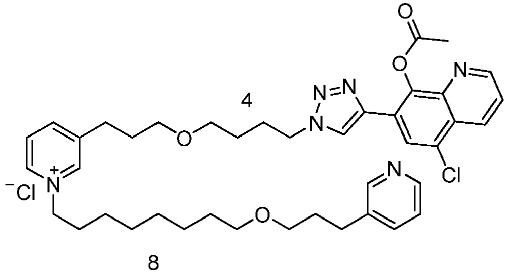

C₄₁H₅₂Cl₂N₆O₄
MM = 762

A partir de pyridine-pyridinium chloride **20** (86 mg, 0.17 mmol) et de l'acetylène TMS-protégé **8** (66 mg, 0.21 mmol), le produit **38** (130 mg, 42%) est obtenu sous la forme d'une huile brune. R*_{f}* = 0.13 (10% MeOH/CH₂Cl₂); ¹H NMR (400 MHz, CDCl₃) *δ* 1.22-1.35 (m, 8H), 1.38 (m, 2H), 1.53 (m, 2H), 1.88 (m, 2H), 1.95-2.05 (m, 6H), 2.59 (s, 3H), 2.70 (m, 2H), 2.96 (m, 2H), 3.35-3.47 (m, 8H), 4.50 (t, *J* = 7.0 Hz, 2H), 4.95 (t, *J* = 7.2 Hz, 2H), 7.23 (m, 1H), 7.51-7.57 (m, 2H), 7.97 (m, 1H), 8.08 (s, 1H), 8.21 (d, *J* = 7.9 Hz, 1H), 8.38-8.45 (m, 2H), 8.52 (s, 1H), 8.55 (dd, *J* = 8.5, 1.6 Hz, 1H), 8.95 (dd, *J* = 4.2, 1.6 Hz, 1H), 9.32-9.37 (m, 2H); ¹³C NMR (100 MHz, CDCl₃) *δ* 21.5 (CH₃), 25.8 (CH₂), 26.3 (2×CH₂), 26.4 (CH₂), 29.2 (CH₂), 29.4 (CH₂), 29.5 (CH₂), 29.7 (CH₂), 29.9 (CH₂), 30.3 (CH₂), 30.4 (CH₂), 31.1 (CH₂), 32.3 (CH₂), 50.7 (CH₂), 62.1 (CH₂), 69.3 (CH₂), 69.6 (CH₂), 70.9 (CH₂), 71.2 (CH₂), 122.6 (CH), 123.2 (CH), 123.6 (CH), 123.9 (C), 125.5 (CH), 126.8 (C), 127.9 (CH), 129.3 (C), 133.3 (CH), 136.3 (CH), 137.6 (C), 141.6 (C), 142.1 (C), 142.6 (C), 142.9 (CH), 144.0 (C), 144.5 (CH), 144.8 (CH), 147.2 (CH), 149.6 (CH), 151.4 (CH), 169.2 (C); LRMS (ESI+) *m*/*z* (%): 727 (2) [*M*⁺] ; 685 (100).

### 3-(3-(6-(4-(8-acetoxy-5-chloroquinolin-7-yl)-1H-1,2,3-triazol-1-yl)hexyloxy)propyl)-1-(6-(3-(pyridin-3-yl)propoxy)hexyl)pyridinium chloride (39)

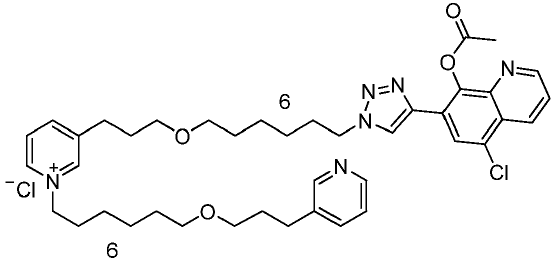

C₄₁H₅₂Cl₂N₆O₄
MM = 762

A partir de pyridine-pyridinium chloride **21** (59 mg, 0.11 mmol) et de l'acetylène TMS-protégé **8** (47 mg, 0.14 mmol), le produit **39** (52 mg, 60%) est obtenu sous la forme d'une huile brune. R*_{f}* = 0.10 (10% MeOH/CH₂Cl₂) ; ¹H NMR (400 MHz, CDCl₃) *δ* 1.33-1.45 (m, 10H), 1.48-1.58 (m, 4H), 1.85 (m, 2H), 1.92-2.09 (m, 4H), 2.59 (s, 3H), 2.68 (t, *J* = 7.5 Hz, 2H), 2.96 (t, *J* = 7.6 Hz, 2H), 3.33-3.45 (m, 8H), 4.50 (t, *J* = 7.0 Hz, 2H), 4.96 (m, 2H), 7.24 (m, 1H), 7.49-7.58 (m, 2H), 8.00 (m, 1H), 8.11 (s, 1H), 8.23 (d, *J* = 7.5 Hz, 1H), 8.45-8.57 (m, 4H), 8.94 (d, *J* = 3.2 Hz, 1H), 9.40-9.45 (m, 2H); ¹³C NMR (100 MHz, CDCl₃) *δ* 21.5 (CH₃), 25.7 (CH₂), 25.8 (CH₂), 26.0 (CH₂), 26.3 (CH₂), 29.4 (CH₂), 29.5 (CH₂), 29.6 (CH₂), 29.8 (CH₂), 30.3 (CH₂), 30.4 (CH₂), 31.0 (CH₂), 32.2 (CH₂), 50.6 (CH₂), 61.8 (CH₂), 69.2 (CH₂), 69.5 (CH₂), 70.6 (CH₂), 70.8 (CH₂), 122.5 (CH), 123.2 (CH), 123.6 (CH), 123.8 (C), 125.3 (CH), 126.7 (C), 127.9 (CH), 129.2 (C), 133.2 (CH), 136.1 (CH), 137.6 (C), 141.5 (C), 142.0 (C), 142.5 (CH), 142.9 (C), 143.9 (C), 144.5 (CH), 144.8 (CH), 147.2 (CH), 149.9 (CH), 151.3 (CH), 169.2 (C); HRMS (ESI+) *m*/*z* (%) : calcd for C₄₁H₅₂ClN₆O₄ [*M*⁻¹] : 727.3733, found: 727.3720.

**3-(3-(6-(4-(8-acetoxy-5-chloroquinolin-7-yl)-1H-1,2,3-triazol-1-yl)hexyloxy)propyl)-1-(8-(3-(pyridin-3-yl)propoxy)octyl)pyridinium chloride (40)** 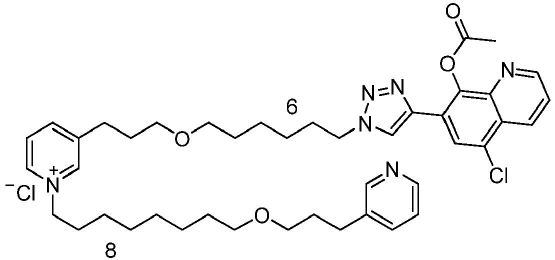 C₄₃H₅₆Cl₂N₆O₄
MM = 790

A partir de pyridine-pyridinium chloride **22** (85.5 mg, 0.16 mmol) et de l'acetylène TMS-protégé **8** (62 mg, 0.20 mmol), le produit **40** (73 mg, 59%) est obtenu sous la forme d'une huile brune. R*_{f}* = 0.17 (10% MeOH/CH₂Cl₂); ¹H NMR (400 MHz, CDCl₃) *δ* 1.20-1.44 (m, 12H), 1.47-1.56 (m, 4H), 1.87 (m, 2H), 1.91-2.09 (m, 6H), 2.59 (s, 3H), 2.69 (t, *J* = 7.5 Hz, 2H), 2.95 (t, *J* = 7.6 Hz, 2H), 3.32-3.45 (m, 8H), 4.50 (t, *J* = 7.0 Hz, 2H), 4.91 (m, 2H), 7.24 (m, 1H), 7.50-7.57 (m, 2H), 7.99 (m, 1H), 8.12 (s, 1H), 8.22 (d, *J* = 7. 0 Hz, 1H), 8.38-8.57 (m, 4H), 8.94 (d, *J* = 3.2 Hz, 1H), 9.28-9.38 (m, 2H); ¹³C NMR (100 MHz, CDCl₃) *δ* 21.4 (CH₃), 25.7 (CH₂), 26.1 (2×CH₂), 26.3 (CH₂), 29.1 (CH₂), 29.2 (CH₂), 29.4 (CH₂), 29.6 (CH₂), 29.7 (CH₂), 29.8 (CH₂), 30.2 (CH₂), 30.3 (CH₂), 31.0 (CH₂), 32.1 (CH₂), 50.6 (CH₂), 61.9 (CH₂), 69.2 (CH₂), 69.5 (CH₂), 70.8 (CH₂), 70.9 (CH₂), 122.5 (CH), 123.3 (CH), 123.6 (CH), 123.8 (C), 125.3 (CH), 126.7 (C), 128.0 (CH), 129.1 (C), 133.1 (CH), 136.4 (CH), 137.6 (C), 141.5 (C), 142.0 (C), 142.5 (CH), 142.8 (C), 143.8 (C), 144.3 (CH), 144.8 (CH), 147.0 (CH), 149.6 (CH), 151.3 (CH), 169.2 (C); LRMS (ESI+) *m*/*z* (%): 755 (2) [*M*]⁺; 713 (100); HRMS (ESI+) *m*/*z* (%): calcd for C₄₃H₅₆ClN₆O₄ [*M*⁺] : 755.4046, found: 755.4029.

### 3-(3-(8-(4-(8-acetoxy-5-chloroquinolin-7-yl)-1H-1,2,3-triazol-1-yl)octyloxy)propyl)-1-(6-(3-(pyridin-3-yl)propoxy)hexyl)pyridinium chloride (41)

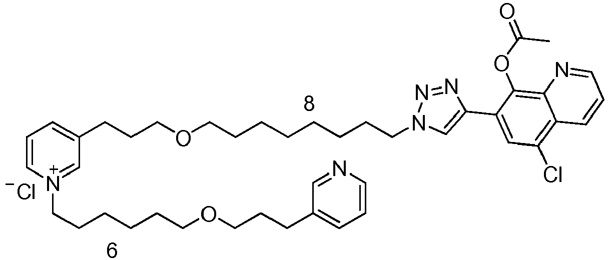

C₄₃H₅₆Cl₂N₆O₄
MM = 790

A partir de pyridine-pyridinium chloride **23** (98.6 mg, 0.18 mmol) et de l'acetylène TMS-protégé **8** (71 mg, 0.22 mmol), le produit **41** (62 mg, 44%) est obtenu sous la forme d'une huile brune. R*_{f}* = 0.14 (10% MeOH/CH₂Cl₂); ¹H NMR (400 MHz, CDCl₃) *δ* 1.22-1.44 (m, 14H), 1.45-1.57 (m, 4H), 1.86 (m, 2H), 1.90-2.09 (m, 6H), 2.57 (s, 3H), 2.70 (m, 2H), 2.95 (m, 2H), 3.30-3.45 (m, 8H), 4.46 (m, 2H), 4.92 (m, 2H), 7.37 (m, 1H), 7.48-7.62 (m, 2H), 7.98 (m, 1H), 8.02 (m, 1H), 8.20 (m, 1H), 8.48-8.57 (m, 2H), 8.94 (s, 1H), 9.25 (m, 1H), 9.34 (m, 1H) ; ¹³C NMR (100 MHz, CDCl₃) *δ* 21.5 (CH₃), 25.8 (CH₂), 26.0 (CH₂), 26.1 (CH₂), 26.5 (CH₂), 29.0 (CH₂), 29.3 (CH₂) , 29.5 (CH₂) , 29.7 (CH₂) , 29.8 (2×CH₂), 30.3 (CH₂), 30.4 (CH₂), 31.0 (CH₂), 32.2 (CH₂), 50.7 (CH₂), 62.0 (CH₂), 69.1 (CH₂), 69.5 (CH₂), 70.6 (CH₂), 71.1 (CH₂), 122.6 (CH), 123.1 (CH), 123.8 (CH), 123.9 (C), 125.5 (CH₂), 126.8 (C), 128.0 (CH), 129.2 (C), 133.3 (CH), 136.3 (CH), 137.6 (C), 141.6 (C), 142.1 (C), 142.5 (CH), 142.9 (C), 144.0 (C), 144.4 (CH), 144.9 (CH), 147.2 (CH), 149.9 (CH), 151.3 (CH), 169.1 (C); HRMS (ESI+) *m*/*z* (%): calcd for C₄₇H₆₄ClN₆O₄ [*M*⁺] : 755.4046, found: 755.4029.

### 3-(3-(10-(4-(8-acetoxy-5-chloroquinolin-7-yl)-1H-1,2,3-triazol-1-yl)decyloxy)propyl)-1-(6-(3-(pyridin-3-yl)propoxy)hexyl)pyridinium chloride (42)

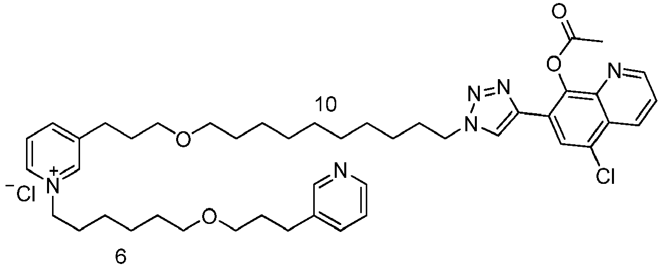

C₄₅H₆₀Cl₂N₆O₄
MM = 818

A partir de pyridine-pyridinium chloride **24** (119 mg, 0.21 mmol) et de l'acetylène TMS-protégé **8** (82 mg, 0.19 mmol), le produit **42** (70 mg, 41%) est obtenu sous la forme d'une huile brune. R*_{f}* = 0.19 (10% MeOH/CH₂Cl₂); ¹H NMR (400 MHz, CDCl₃) *δ* 1.24-1.44 (m, 16H), 1.46-1.57 (m, 4H), 1.86 (m, 2H), 1.93-2.08 (m, 6H), 2.58 (s, 3H), 2.69 (t, *J* = 7.6 Hz, 2H), 2.98 (t, *J* = 7.6 Hz, 2H), 3.33-3.40 (m, 6H), 3.42 (t, *J* = 6.0 Hz, 2H), 4.47 (t, *J* = 7.2 Hz, 2H), 4.97 (t, *J* = 7.2 Hz, 2H), 7.24 (dd, *J* = 7.6, 5.0 Hz, 1H), 7.51-7.57 (m, 2H), 8.00 (m, 1H), 8.04 (s, 1H), 8.22 (d, *J* = 7.8 Hz, 1H), 8.42-8.46 (m, 2H), 8.52 (s, 1H), 8.54 (d, *J* = 8.6 Hz, 1H), 8.94 (dd, *J* = 5.0, 1.5 Hz, 1H), 9.36 (s, 1H), 9.45 (d, *J* = 5.0 Hz, 1H) ; ¹³C NMR (100 MHz, CDCl₃) *δ* 21.4 (CH₃), 25.8 (CH₂), 26.0 (CH₂), 26.2 (CH₂), 26.5 (CH₂), 29.0 (CH₂), 29.4 (2×CH₂), 29.5 (2×CH₂), 29.6 (CH₂), 29.7 (2×CH₂), 30.2 (CH₂), 30.4 (CH₂), 30.9 (CH₂), 32.2 (CH₂), 50.7 (CH₂), 61.8 (CH₂), 69.0 (CH₂), 69.5 (CH₂), 70.6 (CH₂), 71.2 (CH₂), 122.5 (CH), 123.1 (CH), 123.6 (CH), 123.8 (C), 125.4 (CH₂), 126.7 (C), 127.9 (CH), 129.2 (C), 133.2 (CH), 136.4 (CH), 137.5 (C), 141.5 (C), 142.0 (C), 142.5 (CH), 143.0 (C), 143.9 (C), 144.4 (CH), 144.8 (CH), 147.1 (CH), 149.7 (CH), 151.3 (CH), 169.1 (C) ; LRMS (ESI+) *m*/*z* (%) : 783 (1) [*M*⁺] ; 741 (100) .

### 3-(3-(8-(4-(8-acetoxy-5-chloroquinolin-7-yl)-1H-1,2,3-triazol-1-yl)octyloxy)propyl)-1-(8-(3-(pyridin-3-yl)propoxy)octyl)pyridinium chloride (43)

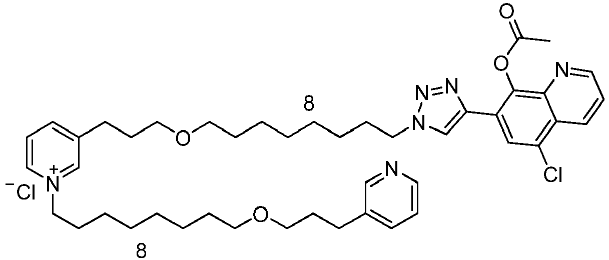

C₄₅H₆₀Cl₂N₆O₄
MM = 818

A partir de pyridine-pyridinium chloride **25** (87 mg, 0.15 mmol) et de l'acetylène TMS-protégé **8** (62 mg, 0.19 mmol), le produit **43** (45 mg, 36%) est obtenu sous la forme d'une huile brune. R*_{f}* = 0.21 (10% MeOH/CH₂Cl₂) ; ¹H NMR (400 MHz, CDCl₃) *δ* 1.22-1.41 (m, 18H), 1.47-1.57 (m, 4H), 1.87 (m, 2H), 1.93-2.07 (m, 6H), 2.58 (s, 3H), 2.70 (t, *J* = 7.6 Hz, 2H), 2.97 (t, *J* = 7.6 Hz, 2H), 3.33-3.45 (m, 8H), 4.48 (m, 2H), 4.96 (m, 2H), 7.29 (dd, *J* = 7.6, 5.0 Hz, 1H), 7.50-7.57 (m, 2H), 7.99 (m, 1H), 8.04 (s, 1H), 8.22 (d, *J* = 7.6 Hz, 1H), 8.41-8.46 (m, 2H), 8.52 (s, 1H), 8.54 (d, *J* = 8.6 Hz, 1H), 8.94 (d, *J* = 5.0 Hz, 1H), 9.36 (s, 1H), 9.43 (d, *J* = 5.0 Hz, 1H) ; ¹³C NMR (100 MHz, CDCl₃) *δ* 21.4 (CH₃), 26.1 (CH₂), 26.2 (2×CH₂), 26.5 (CH₂), 29.0 (CH₂), 29.1 (CH₂), 29.2 (CH₂), 29.3 (CH₂), 29.6 (CH₂), 29.7 (CH₂), 29.8 (2×CH₂), 30.3 (CH₂), 30.4 (CH₂), 31.0 (CH₂), 32.2 (CH₂), 50.7 (CH₂), 61.9 (CH₂), 69.1 (CH₂), 69.5 (CH₂), 71.0 (CH₂), 71.1 (CH₂), 122.5 (CH), 123.1 (CH), 123.6 (CH), 123.9 (C), 125.4 (CH₂), 126.8 (C), 127.9 (CH), 129.2 (C), 133.2 (CH), 136.5 (CH), 137.6 (C), 141.6 (C), 142.1 (C), 142.6 (CH), 142.9 (C), 143.9 (C), 144.5 (CH), 144.8 (CH), 146.9 (CH), 149.6 (CH), 151.3 (CH), 169.2 (C) ; LRMS (ESI+) m/z (%) : 783 (2) [*M*⁺] ; 741 (100) .

### 3-(3-(12-(4-(8-acetoxy-5-chloroquinolin-7-yl)-1H-1,2,3-triazol-1-yl)dodecyloxy)propyl)-1-(4-(3-(pyridin-3-yl)propoxy)butyl)pyridinium chloride (44)

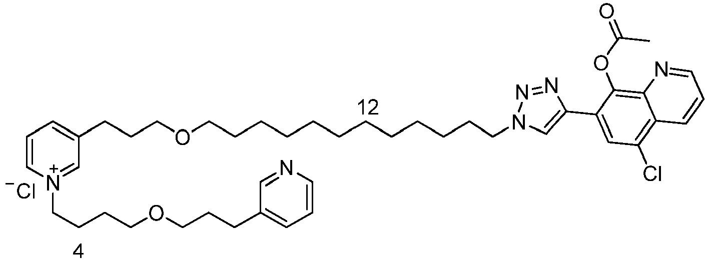

C₄₅H₆₀Cl₂N₆O₄
MM = 818

A partir de pyridine-pyridinium chloride **26** (89 mg, 0.16 mmol) et de l'acetylène TMS-protégé **8** (62 mg, 0.20 mmol), le produit **44** (53 mg, 42%) est obtenu sous la forme d'une huile brune. Rf = 0.26 (10% MeOH/CH₂Cl₂); ¹H NMR (400 MHz, CDCl₃) *δ* 1.17-1.41 (m, 16H), 1.51 (m, 2H), 1.64 (m, 2H), 1.86 (m, 2H), 1.92-2.02 (m, 6H), 2.13 (m, 2H), 2.58 (s, 3H), 2.67 (t, *J* = 7.6 Hz, 2H), 2.98 (t, *J* = 7.6 Hz, 2H), 3.33-3.47 (m, 8H), 4.46 (t, *J* = 7.1 Hz, 2H), 5.01 (t, *J* = 7.0 Hz, 2H), 7.24 (m, 1H), 7.50-7.57 (m, 2H), 7.96-8.04 (m, 2H), 8.22 (d, *J* = 7.7 Hz, 1H), 8.22 (d, *J* = 7.8 Hz, 1H), 8.37-8.49 (m, 2H), 8.52-8.57 (m, 2H), 8.94 (dd, *J* = 4.2, 1.5 Hz, 1H), 9.37 (s, 1H), 9.47 (d, *J* = 5.0 Hz, 1H); ¹³C NMR (100 MHz, CDCl₃) *δ* 21.4 (CH₃), 26.2 (CH₂), 26.3 (CH₂), 26.6 (CH₂), 29.1 (CH₂), 29.4 (2×CH₂), 29.5 (2×CH₂), 29.6 (2×CH₂), 29.8 (3×CH₂), 30.2 (CH₂), 30.4 (CH₂), 30.9 (CH₂), 50.7 (CH₂), 61.6 (CH₂), 69.0 (CH₂), 69.9 (CH₂), 70.0 (CH₂), 71.2 (CH₂), 122.5 (CH), 123.0 (CH), 123.7 (CH), 123.8 (C), 125.4 (CH₂), 126.7 (C), 127.9 (CH), 129.2 (C), 133.2 (CH), 136.3 (CH), 137.5 (C), 141.5 (C), 142.0 (C), 142.4 (CH), 143.0 (C), 143.9 (C), 144.5 (CH), 144.9 (CH), 147.1 (CH), 149.7 (CH), 151.2 (CH), 169.0 (C) ; LRMS (ESI+) *m*/*z* (%): 873 (1) [*M*⁺]; 741 (100).

### 3-(3-(6-(4-(8-acetoxy-5-chloroquinolin-7-yl)-1H-1,2,3-triazol-1-yl)hexyloxy)propyl)-1-(10-(3-(pyridin-3-yl)propoxy)decyl)pyridinium chloride (45)

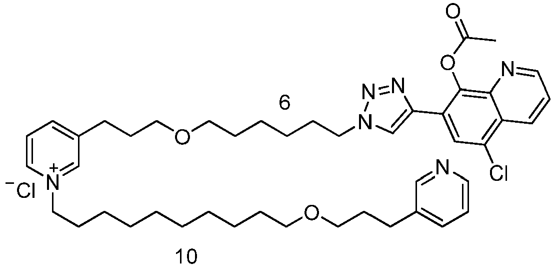

C₄₅H₆₀Cl₂N₆O₄
MM = 818

A partir de pyridine-pyridinium chloride **27** (145 mg, 0.25 mmol) et de l'acetylène TMS-protégé **8** (104 mg, 0.33 mmol), le produit **45** (97 mg, 47%) est obtenu sous la forme d'une huile brune. R*_{f}* = 0.19 (10% MeOH/CH₂Cl₂); ¹H NMR (400 MHz, acetone-d6) δ 1.15-1.41 (m, 16H), 1.42-1.55 (m, 4H), 1.83 (m, 2H), 1.92-2.09 (m, 6H), 2.65 (s, 3H), 2.69 (m, 2H), 2.97 (m, 2H), 3.30-3.44 (m, 8H), 4.66 (m, 2H), 4.97 (m, 2H), 7.29 (m, 1H), 7.61 (d, *J* = 7.6 Hz, 1H), 7.71 (m, 1H), 8.11 (s, 1H), 8.40-8.66 (m, 5H), 8.97-9.04 (m, 2H), 9.45 (m, 1H), 9.68 (m, 1H) ; ¹³C NMR (100 MHz, CDCl₃) δ 21.5 (CH₃), 25.8 (CH₂), 26.3 (2×CH₂), 26.4 (CH₂), 29.2 (CH₂), 29.5 (2×CH₂), 29.6 (2×CH₂), 29.7 (2×CH₂), 29.9 (CH₂), 30.3 (CH₂), 30.4 (CH₂), 31.2 (CH₂), 32.2 (CH₂), 50.7 (CH₂), 62.1 (CH₂), 69.2 (CH₂), 69.6 (CH₂), 70.8 (CH₂), 71.2 (CH₂), 122.6 (CH), 123.2 (CH), 123.9 (CH, C), 125.5 (CH) , 126.8 (C), 127.9 (CH), 129.3 (C), 133.3 (CH), 136.1 (CH), 137.7 (C), 141.6 (C), 142.1 (C), 142.6 (CH), 142.8 (C), 144.0 (C), 144.5 (CH), 144.9 (CH), 147.2 (CH), 149.9 (CH), 151.4 (CH), 169.2 (C) ; LRMS (ESI+) *m*/*z* (%) : 783 (1) [*M*⁺]; 741 (100) .

### 3-(3-(6-(4-(8-acetoxy-5-chloroquinolin-7-yl)-1H-1,2,3-triazol-1-yl)hexyloxy)propyl)-1-(12-(3-(pyridin-3-yl)propoxy)dodecyl)pyridinium chloride (46)

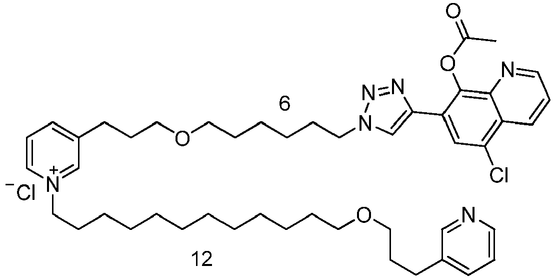

C₄₇H₆₄Cl₂N₆O₄
MM = 846

A partir de pyridine-pyridinium chloride **28** (89 mg, 0.15 mmol) et de l'acetylène TMS-protégé **8** (59 mg, 0.18 mmol), le produit **46** (42 mg, 34%) est obtenu sous la forme d'une huile brune. R*_{f}* = 0.12 (10% MeOH/CH₂Cl₂); ¹H NMR (400 MHz, CDCl₃) δ 1.20-1.40 (m, 20H), 1.46-1.64 (m, 4H), 1.88 (m, 2H), 1.95-2.10 (m, 6H), 2.61 (s, 3H), 2.71 (m, 2H), 2.98 (t, *J* = 7.6 Hz, 2H), 3.32-3.48 (m, 8H), 4.55 (t, *J* = 7.0 Hz, 2H), 4.94 (m, 2H), 7.27 (m, 1H), 7.52-7.60 (m, 2H), 7.94 (m, 1H), 8.16 (s, 1H), 8.22 (d, *J* = 7.0 Hz, 1H), 8.40-8.58 (m, 4H), 8.95 (d, *J* = 3.2 Hz, 1H), 9.23 (m, 1H), 9.27 (m, 1H) ; ¹³C NMR (100 MHz, CDCl₃) δ 21.6 (CH₃), 26.2 (2×CH₂), 26.7 (CH₂), 27.7(CH₂), 29.2 (CH₂), 29.3 (2×CH₂), 29.7 (3×CH₂), 29.8 (3×CH₂), 29.9 (CH₂), 30.2 (CH₂), 31.0 (2×CH₂), 32.3 (CH₂), 50.6 (CH₂), 62.1 (CH₂), 69.4 (CH₂), 69.5 (CH₂), 70.1 (CH₂), 71.1 (CH₂), 122.6 (CH), 123.5 (CH), 123.9 (CH, C), 125.4 (CH), 126.9 (C), 127.9 (CH), 129.3 (C), 133.3 (CH), 136.9 (CH), 137.9 (C), 141.6 (C), 142.1 (C), 142.6 (CH, C), 144.0 (C), 144.8 (2×CH), 144.8 (CH), 146.7 (CH), 149.3 (CH), 151.4 (CH), 169.4 (C) ; HRMS (ESI+): calcd for C₄₇H₆₄ClN₆O₄ [*M*⁺] : 811.4672, found: 811.4675.

### 3-(3-(8-(4-(8-acetoxy-5-chloroquinolin-7-yl)-1H-1,2,3-triazol-1-yl)octyloxy)propyl)-1-(10-(3-(pyridin-3-yl)propoxy)decyl)pyridinium chloride (47)

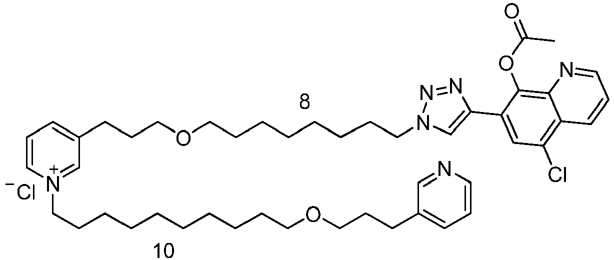

C₄₇H₆₄Cl₂N₆O₄
MM = 846

A partir de pyridine-pyridinium chloride **29** (27 mg, 0.045 mmol) et de l'acetylène TMS-protégé **8** (14 mg, 0.018 mmol), le produit **47** (29 mg, 77%) est obtenu sous la forme d'une huile brune. R*_{f}* = 0.30 (10% MeOH/CH₂Cl₂); ¹H NMR (400 MHz, CDCl₃) δ 1.17-1.42 (m, 22H), 1.47-1.63 (m, 4H), 1.89 (m, 2H), 1.94-2.07 (m, 6H), 2.59 (s, 3H), 2.72 (m, 2H), 2.99 (m, 2H), 3.33-3.48 (m, 8H), 4.49 (t, *J* = 7.1 Hz, 2H), 4.94 (m, 2H), 7.27 (m, 1H), 7.51-7.59 (m, 2H), 8.01 (m, 1H), 8.06 (s, 1H), 8.25 (d, *J* = 7.2 Hz, 1H), 8.50-8.58 (m, 2H), 8.95 (m, 1H), 9.24-9.37 (m, 2H) ; ¹³C NMR (100 MHz, CDCl₃) δ 21.5 (CH₃), 26.1 (CH₂), 26.2 (CH₂), 26.3 (CH₂), 26.5 (CH₂), 28.9 (CH₂), 29.2 (CH₂), 29.0 (CH₂), 29.2 (CH₂), 29.5 (CH₂), 29.6 (3×CH₂), 29.7 (CH₂), 29.8 (CH₂), 29.9 (CH₂), 30.3 (CH₂), 30.4 (CH₂), 31.1 (CH₂), 32.2 (CH₂), 50.7 (CH₂), 62.0 (CH₂), 69.1 (CH₂), 69.5 (CH₂), 71.1 (CH₂), 71.2 (CH₂), 122.5 (CH), 123.2 (CH), 123.9 (CH, C), 125.4 (CH₂), 126.8 (C), 128.0 (CH), 129.2 (C), 133.2 (CH), 136.3 (CH), 137.6 (C), 141.6 (C), 142.1 (C), 142.5 (CH), 142.7 (C), 143.9 (C), 144.3 (CH), 145.1 (CH), 147.2 (CH), 149.8 (CH), 151.3 (CH), 169.1 (C) ; LRMS (ESI+) *m*/*z* (%) : 811 (1) [*M*⁺]; 769 (100) .

### 3-(3-(10-(4-(8-acetoxy-5-chloroquinolin-7-yl)-1H-1,2,3-triazol-1-yl)decyloxy)propyl)-1-(10-(3-(pyridin-3-yl)propoxy)decyl)pyridinium chloride (48)

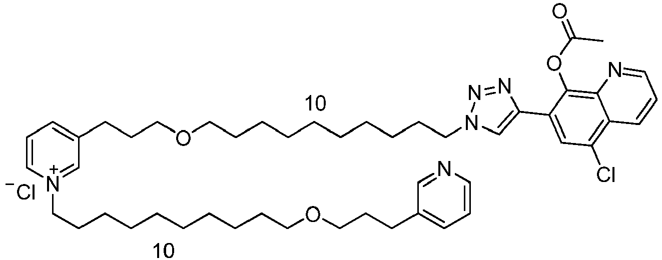

C₄₉H₆₈Cl₂N₆O₄
MM = 874

A partir de pyridine-pyridinium chloride **30** (130 mg, 0.21 mmol) et de l'acetylène TMS-protégé **8** (85 mg, 0.27 mmol), le produit **48** (110 mg, 61%) est obtenu sous la forme d'une huile brune. R*_{f}* = 0.33 (10% MeOH/CH₂Cl₂); ¹H NMR (400 MHz, CDCl₃) δ 1.18-1.40 (m, 24H), 1.46-1.60 (m, 4H), 1.88 (m, 2H), 1.93-2.06 (m, 6H), 2.58 (s, 3H), 2.70 (t, *J* = 7.6 Hz, 2H), 2.98 (t, *J* = 7.6 Hz, 2H), 3.32-3.44 (m, 8H), 4.47 (t, *J* = 7.2 Hz, 2H), 4.94 (m, 2H), 7.23 (dd, *J* = 7.6, 5.0 Hz, 1H), 7.51-7.54 (m, 2H), 7.98-8.09 (m, 2H), 8.23 (d, *J* = 7.7 Hz, 1H), 8.40-8.50 (m, 2H), 8.51-8.56 (m, 2H), 8.94 (dd, *J* = 5.0, 1.5 Hz, 1H), 9.34 (s, 1H), 9.42 (m, 1H) ; ¹³C NMR (100 MHz, CDCl₃) δ 21.3 (CH₃), 26.1 (2×CH₂), 26.2 (CH₂), 26.5 (CH₂), 28.9 (CH₂), 29.1 (CH₂), 29.3 (CH₂), 29.4 (2×CH₂), 29.5 (3×CH₂), 29.6 (CH₂), 29.7 (2×CH₂), 29.8 (CH₂), 30.2 (CH₂), 30.4 (CH₂), 31.0 (CH₂), 32.1 (CH₂), 50.6 (CH₂), 61.8 (CH₂), 68.9 (CH₂), 69.4 (CH₂), 71.0 (CH₂), 71.1 (CH₂), 122.4 (CH), 123.0 (CH), 123.5 (CH), 123.8 (C), 125.3 (CH₂), 126.6 (C), 127.9 (CH), 129.1 (C), 133.1 (CH), 136.3 (CH), 137.5 (C), 141.4 (C), 142.0 (C), 142.4 (CH), 142.9 (C), 143.8 (C), 144.3 (CH), 144.8 (CH), 147.0 (CH), 149.6 (CH), 151.2 (CH), 169.0 (C) ; LRMS (ESI+) *m*/*z* (%): 839 (1) [*M*⁺]; 811 (100).

### 3-(3-(12-(4-(8-acetoxy-5-chloroquinolin-7-yl)-1H-1,2,3-triazol-1-yl)dodecyloxy)propyl)-1-(8-(3-(pyridin-3-yl)propoxy)octyl)pyridinium chloride (49)

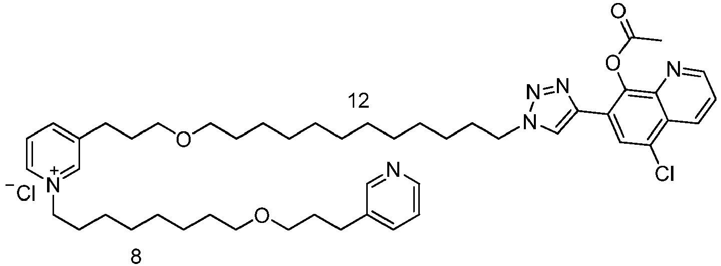

C₄₉H₆₈Cl₂N₆O₄
MM = 874

A partir de pyridine-pyridinium chloride **31** (109 mg, 0.17 mmol) et de l'acetylène TMS-protégé **8** (68 mg, 0.19 mmol), le produit **49** (95 mg, 63%) est obtenu sous la forme d'une huile brune. R*_{f}* = 0.25 (10% MeOH/CH₂Cl₂); ¹H NMR (400 MHz, CDCl₃) δ 1.20-1.40 (m, 24H), 1.46-1.57 (m, 4H), 1.88 (m, 2H), 1.92-2.07 (m, 6H), 2.58 (s, 3H), 2.70 (t, *J* = 7.6 Hz, 2H), 2.98 (t, *J* = 7.6 Hz, 2H), 3.32-3.46 (m, 8H), 4.46 (t, *J* = 7.1 Hz, 2H), 4.95 (m, 2H), 7.24 (m, 1H), 7.50-7.57 (m, 2H), 7.96-8.04 (m, 2H), 8.23 (d, *J* = 7.7 Hz, 1H), 8.42-8.53 (m, 2H), 8.53-8.58 (m, 2H), 8.94 (m, 1H), 9.30 (s, 1H), 9.42 (m, 1H); ¹³C NMR (100 MHz, CDCl₃) δ 21.4 (CH₃), 26.1 (2×CH₂), 26.2 (CH₂), 26.6 (CH₂), 29.0 (CH₂), 29.2 (CH₂), 29.4 (CH₂), 29.5 (2×CH₂), 29.6 (3×CH₂), 29.7 (3×CH₂), 30.2 (CH₂), 30.4 (CH₂), 31.0 (CH₂), 32.1 (CH₂), 50.7 (CH₂), 61.9 (CH₂), 69.0 (CH₂), 69.5 (CH₂), 70.1 (CH₂), 71.2 (CH₂), 122.5 (CH), 123.0 (CH), 123.8 (CH, C), 125.4 (CH₂), 126.7 (C), 127.9 (CH), 129.1 (C), 133.2 (CH), 136.2 (CH), 137.5 (C), 141.5 (C), 142.0 (C), 142.4 (CH), 143.0 (C), 143.8 (C), 144.3 (CH), 144.9 (CH), 147.1 (CH), 149.8 (CH), 151.2 (CH), 169.0 (C) ; LRMS (ESI+) *m*/*z* (%): 839 (1) [*M*]⁺; 797 (100) ; HRMS (ESI+) *m*/*z* (%) : calcd for C₄₉H₆₈ClN₆O₄ [*M*⁺] : 839.4985, found: 839.4879.

### 3-(3-(10-(4-(8-acetoxy-5-chloroquinolin-7-yl)-1H-1,2,3-triazol-1-yl)decyloxy)propyl)-1-(12-(3-(pyridin-3-yl)propoxy)dodecyl)pyridinium chloride (50)

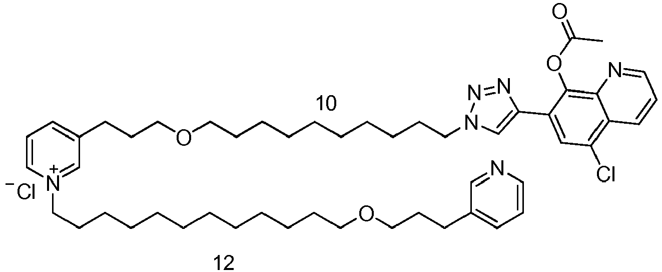

C₅₁H₇₂Cl₂N₆O₄
MM = 902

A partir de pyridine-pyridinium chloride **32** (86 mg, 0.17 mmol) et de l'acetylène TMS-protégé **8** (66 mg, 0.21 mmol), le produit **50** (54 mg, 42%) est obtenu sous la forme d'une huile brune. R*_{f}* = 0.28 (10% MeOH/CH₂Cl₂); ¹H NMR (400 MHz, CDCl₃) δ 1.17-1.42 (m, 28H), 1.46-1.52 (m, 4H), 1.88 (m, 2H), 1.92-2.07 (m, 6H), 2.58 (s, 3H), 2.70 (m, 2H), 2.98 (t, *J* = 7.6 Hz, 2H), 3.33-3.46 (m, 8H), 4.47 (t, *J* = 7.1 Hz, 2H), 4.93 (t, *J* = 7.0 Hz, 2H), 7.23 (m, 1H), 7.54 (m, 2H), 7.97-8.06 (m, 2H), 8.22 (d, *J* = 7.7 Hz, 1H), 8.40-8.48 (m, 2H), 8.51-8.58 (m, 2H), 8.94 (dd, *J* = 5.0, 1.5 Hz, 1H), 9.28 (s, 1H), 9.39 (m, 1H); ¹³C NMR (100 MHz, CDCl₃) δ 21.4 (CH₃), 26.2 (2×CH₂), 26.3 (CH₂), 26.5 (CH₂), 29.0 (CH₂), 29.2 (CH₂), 29.4 (4×CH₂), 29.5 (2×CH₂), 29.6 (CH₂), 29.7 (2×CH₂), 29.8 (3×CH₂), 30.2 (CH₂), 30.4 (CH₂), 31.1 (CH₂), 32.2 (CH₂), 50.7 (CH₂), 62.0 (CH₂), 69.0 (CH₂), 69.5 (CH₂), 71.1 (CH₂), 71.2 (CH₂), 122.5 (CH), 123.1 (CH), 123.5 (CH), 123.9 (C), 125.4 (CH₂), 126.7 (C), 128.0 (CH), 129.2 (C), 133.2 (CH), 136.2 (CH), 137.5 (C), 141.5 (C), 142.0 (C), 142.5 (CH), 142.9 (C), 143.8 (C), 144.3 (CH), 144.9 (CH), 147.2 (CH), 149.9 (CH), 151.3 (CH), 169.1 (C) ; LRMS (ESI+) *m*/*z* (%) : 867 (1) [*M*⁺]; 825 (100) .

### 3-(3-(12-(4-(8-acetoxy-5-chloroquinolin-7-yl)-1H-1,2,3-triazol-1-yl)dodecyloxy)propyl)-1-(10-(3-(pyridin-3-yl)propoxy)decyl)pyridinium chloride (51)

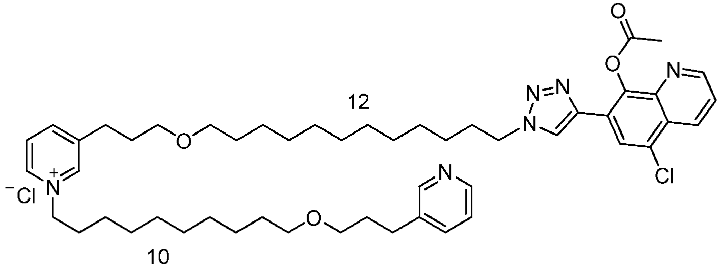

C₅₁H₇₂Cl₂N₆O₄
MM = 904

A partir de pyridine-pyridinium chloride **33** (71 mg, 0.11 mmol) et de l'acetylène TMS-protégé **8** (44 mg, 0.14 mmol), le produit **51** (50 mg, 51%) est obtenu sous la forme d'une huile brune.R*_{f}* = 0.22 (10% MeOH/CH₂Cl₂); ¹H NMR (400 MHz, CDCl₃) δ 1.19-1.40 (m, 28H), 1.48-1.61 (m, 4H), 1.89 (m, 2H), 1.94-2.05 (m, 6H), 2.58 (s, 3H), 2.70 (m, 2H), 2.99 (t, *J* = 7.6 Hz, 2H), 3.34-3.47 (m, 8H), 4.46 (t, *J* = 7.1 Hz, 2H), 4.95 (m, 2H), 7.23 (m, 1H), 7.50-7.58 (m, 2H), 7.96-8.03 (m, 2H), 7.96-8.03 (m, 2H), 8.22 (d, *J* = 7.7 Hz, 1H), 8.42-8.52 (m, 2H), 8.53-8.59 (m, 2H), 8.95 (m, 1H), 9.23 (s, 1H), 9.38 (m, 1H); ¹³C NMR (100 MHz, CDCl₃) δ 21.4 (CH₃), 26.3 (3×CH₂), 26.6 (CH₂), 29.1 (CH₂), 29.2 (CH₂), 29.5 (2×CH₂), 29.7 (6×CH₂), 29.8 (3×CH₂), 29.9 (CH₂), 30.3 (CH₂), 30.5 (CH₂), 31.1 (CH₂), 32.2 (CH₂), 50.8 (CH₂), 62.1 (CH₂), 69.0 (CH₂), 69.6 (CH₂), 71.2 (CH₂), 71.3 (CH₂), 122.5 (CH), 123.0 (CH), 123.7 (CH), 123.9 (C), 125.5 (CH₂), 126.8 (C), 128.0 (CH), 129.3 (C), 133.3 (CH), 136.2 (CH), 137.6 (C), 141.6 (C), 142.1 (C), 142.5 (CH), 143.0 (C), 143.9 (C), 144.3 (CH), 144.9 (CH), 147.2 (CH), 149.9 (CH), 151.3 (CH), 169.1 (C) ; LRMS (ESI+) *m*/*z* (%) : 867 (1) [*M*⁺]; 825 (100) .

### 3-(3-(12-(4-(8-acetoxy-5-chloroquinolin-7-yl)-1H-1,2,3-triazol-1-yl)dodecyloxy)propyl)-1-(12-(3-(pyridin-3-yl)propoxy)dodecyl)pyridinium chloride (52)

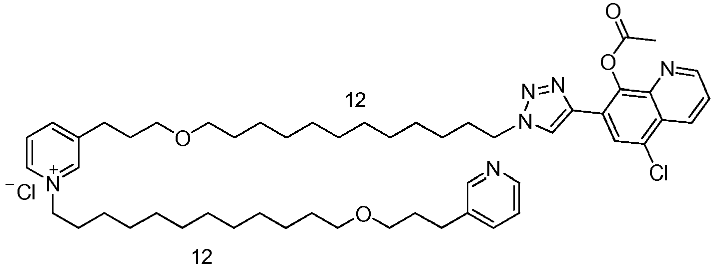

C₅₃H₇₆Cl₂N₆O₄
MM = 930

A partir de pyridine-pyridinium chloride **34** (84 mg, 0.12 mmol) et de l'acetylène TMS-protégé **8** (50.5 mg, 0.16 mmol), le produit **52** (79 mg, 70%) est obtenu sous la forme d'une huile brune. R*_{f}* = 0.26 (10% MeOH/CH₂Cl₂); ¹H NMR (400 MHz, CDCl₃) δ 1.18-1.41 (m, 32H), 1.47-1.59 (m, 4H), 1.88 (m, 2H), 1.92-2.07 (m, 6H), 2.58 (s, 3H), 2.77 (m, 2H), 2.98 (m, 2H), 3.33-3.45 (m, 8H), 4.47 (t, *J* = 7.1 Hz, 2H), 4.93 (m, 2H), 7.50-7.68 (m, 3H), 7.97-8.09 (m, 2H), 8.23 (d, *J* = 6.7 Hz, 1H), 8.39-8.51 (m, 2H), 8.51-8.58 (m, 2H), 8.94 (m, 1H), 9.28 (s, 1H), 9.39 (m, 1H) ; ¹³C NMR (100 MHz, CDCl₃) δ 21.3 (CH₃), 26.2 (3×CH₂), 26.5 (CH₂), 29.0 (CH₂), 29.1 (CH₂), 29.3 (CH₂), 29.4 (2×CH₂), 29.5 (5×CH₂), 29.6 (CH₂), 29.7 (CH₂), 29.8 (2×CH₂), 30.2 (CH₂), 30.4 (CH₂), 31.1 (CH₂), 32.1 (CH₂), 50.7 (CH₂), 62.0 (CH₂), 69.0 (CH₂), 69.5 (CH₂), 71.1 (CH₂), 71.2 (CH₂), 122.4 (CH), 123.0 (CH), 123.8 (CH, C), 125.4 (CH₂), 126.7 (C), 127.9 (CH), 129.1 (C), 133.3 (CH), 135.3 (CH), 135.5 (C), 141.5 (C), 142.0 (C), 142.4 (CH), 142.9 (C), 143.8 (C), 144.3 (CH), 144.9 (CH), 147.2 (CH), 149.8 (CH), 151.2 (CH), 169.0 (C) ; LRMS (ESI+) *m*/*z* (%) : 895 (1) [*M*⁺]; 853 (100).

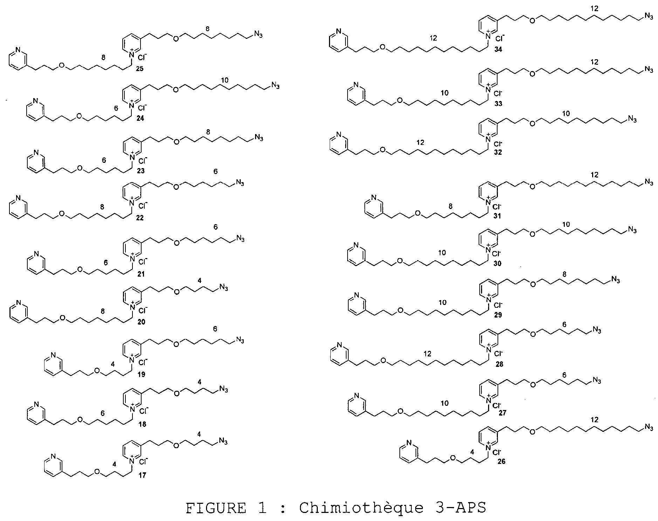

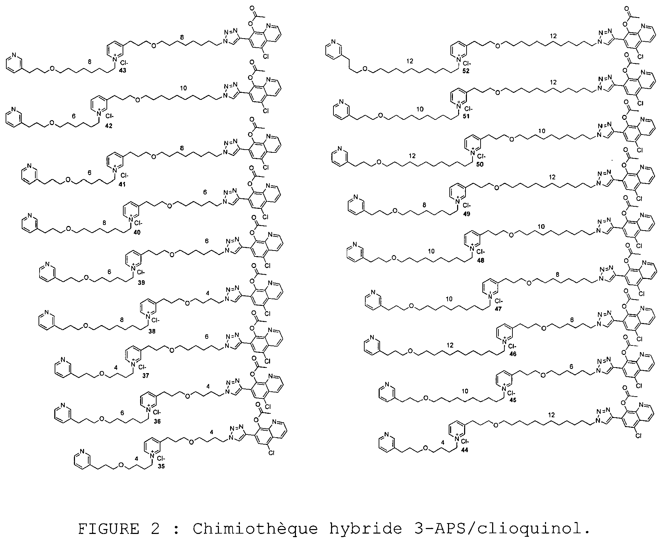

## Revendications

1. Procédé d'identification d'une molécule inhibitrice de la machinerie de virulence de Pseudomonas aeruginosa comprenant une étape d'évaluation de la capacité de ladite molécule à inhiber l'interaction PscE/PscG.

2. Procédé selon la revendication 1, **caractérisé en ce que** l'évaluation de la capacité de ladite molécule à inhiber l'interaction PscE/PscG est réalisée par une technique ELISA.

3. Procédé selon la revendication 1 ou 2 comprenant les étapes suivantes :
a) Fournir un support sur lequel est fixée une des deux molécules PscE ou PscG,
b) Mettre en contact le support avec une solution contenant la molécule PscE ou PscG, la molécule en solution correspondant à celle non fixée au support,
c) Ajouter une solution contenant la molécule à tester
d) Laver le support
e) Ajouter un anticorps primaire dirigé contre la molécule non fixée au support, Laver le support
f) Ajouter un anticorps secondaire dirigé contre le premier anticorps et couplé à une enzyme,
g) Laver le support
h) Ajouter un substrat de l'enzyme générant lors de son hydrolyse par l'enzyme un signal,
i) Quantification du signal
j) Evaluation de la capacité d'inhibition de la molécule à tester par comparaison avec une gamme étalon.

4. Composé de formule (A) : 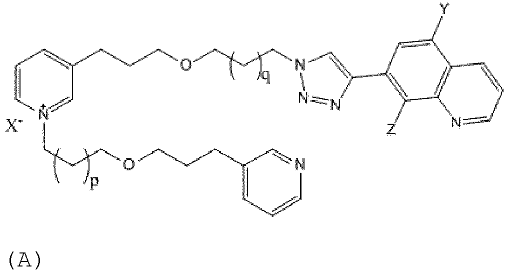 **caractérisé en ce que** :
X est un halogène ;
Y est un halogène ;
Z est un groupement hydroxy, amine, ou un groupement -OR1 dans lequel R1 est un alkyle en C1-C4 ou un acyle en C1-C4 ;
p est un nombre entier et 13 > p ≥ 2 ; et
q est un nombre entier et 13 > q ≥ 2, préférentiellement p+q <23 ;
à la condition que :
si p =4, alors q ≠ 8 ;
si p =10, alors q ≠ 4 ; et
si p =8, alors q ≠ 6 .

5. Composé de formule (A) selon la revendication 4 **caractérisé en ce que** :
- Z est -OH ou OAc ;
- Y est Cl ;
- X est Cl ; et
- p et q sont des nombres pairs avec préférentiellement 8 ≥ p ≥ 2 et 10 ≥ q ≥ 2.

6. Composé de formule (A) selon la revendication 4 ou 5 **caractérisé en ce que** :
- p =2 et q =2, 4 ou 10, préférentiellement 4 ;
- p =4 et q =2, 4 ou 6, préférentiellement 4 ;
- p =6 et q =2, 4, 6 ou 10, préférentiellement 4 ; ou
- p =8 et q =4 ou 8, préférentiellement 4.

7. Procédé de fabrication de composé de formule (A) **caractérisé en ce que** le composé de formule B : 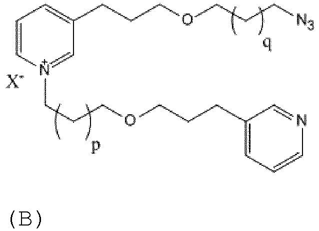 dans lequel X, p et q sont tels que définis dans l'une quelconque des revendications 4 à 6,
est mis en réaction en présence d'un catalyseur, préférentiellement du cuivre, avec le composé de formule C : 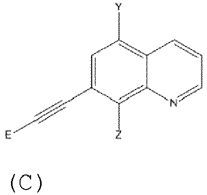 dans lequel Y et Z sont tels que définis dans l'une quelconque des revendications 4 à 6,
et E est un groupement partant choisi dans le groupe constituté par le triméthylesilyle, le tri-isopropyl silyle (TIPS) et dimethyl alcool.

8. Composé de formule (D) : 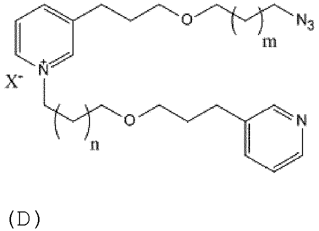 **caractérisé en ce que** :
X est un halogène ;
m est un nombre entier et 13 > m > 2 ; et
n est un nombre entier et 13 > n ≥ 2, préférentiellement 23 > m+n, et les sels pharmaceutiquement acceptables.

9. Composé de formule (D) selon la revendication 8 **caractérisé en ce que** :
- X est Cl ;
- m et n sont des nombres pairs ; et/ou
- préférentiellement m+n > 6

10. Composé de formule (D) selon la revendication 8 ou 9 **caractérisé en ce que** :
- m =4 et n =6 ou 8, préférentiellement 6 ;
- m =6 et n =8 ou 10, préférentiellement 10 ; ou
- m =8 et n =4, 6, 8, ou 10, préférentiellement 6 ou 10,
- m =10 et n= 4 ou 10, préférentiellement 10.

11. Composés de formule (A) selon l'une des revendications 4 à 6 et/ou (D) selon l'une des revendications 8 ou 9 en tant que médicament.

12. Composés de formule (A) selon l'une des revendications 4 à 6 et/ou (D) selon l'une des revendications 11 ou 12 pour leur utilisation pour prévenir et/ou traiter une infection pathogène causée par Pseudomonas aeruginosa.

13. Composés de formule (A) selon l'une des revendications 4 à 6 et/ou (D)) selon l'une des revendications 8 ou 9 pour leur utilisation selon la revendication 12 **caractérisée en ce que** l'infection pathogène est une infection nosocomiale, préférentiellement choisie parmi les infections des yeux, des plaies, en particulier suite à une brûlure et/ou une opératoire, des urines, en particulier de la vessie ou de l'urètre par exemple après sondage, du système gastro-intestinale, d'un poumon en particulier après bronchoscopie, des méningites en particulier d'inoculation, des septicémies.

## Patentansprüche

1. Verfahren zur Identifikation eines auf die Virulenzmaschinerie von Pseudomonas aeruginosa inhibitorisch wirkenden Moleküls, umfassend einen Schritt der Evaluierung der Kapazität des Moleküls, die Interaktion von PscE/PscG zu hemmen.

2. Verfahren nach Anspruch 1, **dadurch gekennzeichnet, dass** die Evaluierung der Kapazität des Moleküls, die Interaktion von PscE/PscG zu hemmen, durch eine ELISA-Technik realisiert wird.

3. Verfahren nach Anspruch 1 oder 2, die folgenden Schritte umfassend:
a) Liefern eines Trägers, an dem eines der zwei Moleküle PscE oder PscG fixiert ist,
b) Inkontaktbringen des Trägers mit einer Lösung, die das Molekül PscE oder PscG enthält, wobei das Molekül in Lösung dem nicht an dem Träger befestigten Molekül entspricht,
c) Hinzufügen einer Lösung, die das zu testende Molekül enthält,
d) Waschen des Trägers,
e) Hinzufügen eines primären Antikörpers, der gegen das nicht an dem Träger befestigte Molekül gerichtet ist, Waschen des Trägers,
f) Hinzufügen eines sekundären Antikörpers, der gegen den primären Antikörper gerichtet ist und an ein Enzym gekoppelt ist,
g) Waschen des Trägers,
h) Hinzufügen eines Substrats des Enzyms, das bei seiner Pyrolyse durch das Enzym ein Signal erzeugt,
i) Quantifizieren des Signals,
j) Evaluieren der Hemmungskapazität des zu testenden Moleküls durch Vergleich mit einer Standardreihe.

4. Verbindung der Formel (A): 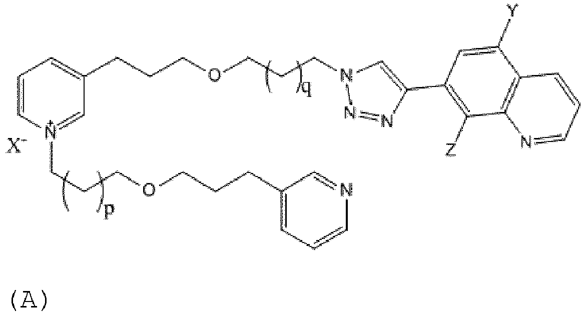 **dadurch gekennzeichnet, dass**:
X ein Halogen ist;
Y ein Halogen ist;
Z eine Hydroxy-, Amingruppe oder eine -OR1 Gruppe ist, in der R1 ein C1-C4-Alkyl oder ein C1-C4-Acyl ist;
wobei p eine ganze Zahl ist und 13 > p ≥ 2; und
q eine ganze Zahl ist und 13 > q ≥ 2, vorzugsweise p + q < 23;
unter der Bedingung dass:
wenn p = 4, dann q ≠ 8;
wenn p = 10, dann q ≠ 4; und
wenn p = 8, dann q ≠ 6.

5. Verbindung der Formel (A) nach Anspruch 4, **dadurch gekennzeichnet, dass**:
- Z ist -OH oder OAc;
- Y ist Cl;
- X ist Cl; und
- p und q sind gerade Zahlen mit vorzugsweise 8 ≥ p ≥ 2 und 10 ≥ q ≥ 2.

6. Verbindung der Formel (A) nach Anspruch 4 oder 5, **dadurch gekennzeichnet, dass**:
- p = 2 und q = 2, 4 oder 10; vorzugsweise 4;
- p = 4 und q = 2, 4 oder 6, vorzugsweise 4;
- p = 6 und q = 2, 4, 6 oder 10, vorzugsweise 4; oder
- p = 8 und q = 4 oder 8, vorzugsweise 4.

7. Verfahren der Herstellung der Verbindung der Formel (A), **dadurch gekennzeichnet, dass** die Verbindung der Formel (B): 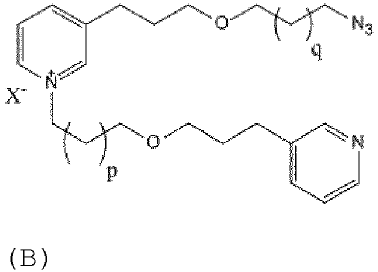 bei der X, p und q so definiert sind wie in einem der Ansprüche 4 bis 6, in Gegenwart eines Katalysators, vorzugsweise Kupfer, mit der Verbindung der Formel (C) umgesetzt wird: 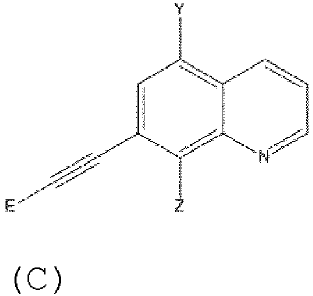 bei der Y und Z so wie in einem der Ansprüche 4 bis 6 definiert sind,
und E eine Abgangsgruppe ausgewählt aus der Gruppe bestehend aus Trimethylesilyl, Tri-Isopropylsilyl (TIPS) und Dimethylalkohol ist.

8. Verbindung der Formel (D): 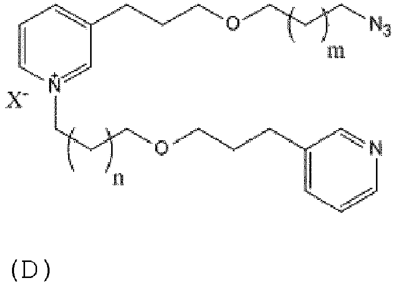 **dadurch gekennzeichnet, dass**:
X ein Halogen ist;
m eine ganze Zahl und 13 > m > 2 ist; und
n eine ganze Zahl und 13 > n ≥ n, vorzugsweise 23 > m + n, und pharmazeutisch annehmbare Salze.

9. Verbindung der Formel (D) nach Anspruch 8, **dadurch gekennzeichnet, dass**:
- X Cl ist;
- m und n gerade Zahlen sind; und/oder
- vorzugsweise m + n > 6.

10. Verbindung von Formel (D) nach Anspruch 8 oder 9, **dadurch gekennzeichnet, dass**:
- m = 4 und n = 6 oder 8, vorzugsweise 6;
- m = 6 und n = 8 oder 10, vorzugsweise 10; oder
- m = 8 und n = 4, 6, 8 oder 10; vorzugsweise 6 oder 10,
- m = 10 und n = 4 oder 10, vorzugsweise 10.

11. Verbindung der Formel (A) nach einem der Ansprüche 4 bis 6 und/oder (D) nach einem der Ansprüche 8 oder 9, als Medikament.

12. Verbindung der Formel (A) nach einem der Ansprüche 4 bis 6 und/oder (D) nach einem der Ansprüche 8 oder 9, für ihre Verwendung zum Verhindern und/oder Behandeln einer pathogenen Infektion, die durch die Pseudomonas aeruginosa verursacht wurde.

13. Verbindung der Formel (A) nach einem der Ansprüche 4 bis 6 und/oder (D) nach einem der Ansprüche 8 oder 9, für ihre Verwendung nach Anspruch 12, **dadurch gekennzeichnet, dass** die pathogenen Infektion eine nosokomiale Infektion ist, vorzugsweise ausgewählt aus Infektionen der Augen, von Wunden, insbesondere als Folge von Verbrennungen und/oder einer Operation, der Harnsysteme, insbesondere der Harnblase oder der Harnröhre nach einer Katheterisierung, des gastrointestinalen Systems, einer Lunge, insbesondere nach einer Bronchoskopie, der Meningitiden, insbesondere der Beimpfung, von Blutvergiftungen.

## Claims

1. A process for identifying a molecule which inhibits the virulence machinery of *Pseudomonas aeruginosa* comprising a step of evaluating the capacity of said molecule to inhibit the PscE/PscG interaction.

2. The process according to claim 1, **characterized in that** the evaluation of the capacity of said molecule to inhibit the PscE/PscG interaction is carried out by an ELISA technique.

3. The process according to claim 1 or 2 comprising the following steps:
a) Provide a support onto which is fixed one of the two molecules PscE or PscG,
b) Contact the support with a solution containing the molecule PscE or PscG, the molecule in solution corresponding to the one not fixed onto the support,
c) Add a solution containing the molecule to be tested
d) Wash the support
e) Add a primary antibody against the molecule not fixed onto the support, Wash the support
f) Add a secondary antibody against the primary antibody and coupled to an enzyme,
g) Wash the support
h) Add a substrate for the enzyme which generates a signal during its hydrolysis by the enzyme,
i) Quantify the signal
j) Evaluate the inhibition capacity of the molecule to be tested by comparison with a standard range.

4. A compound of formula (A) : 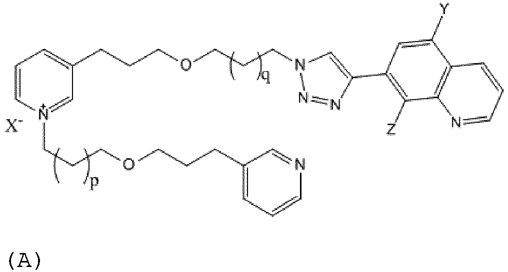 **characterized in that**:
X is a halogen;
Y is a halogen;
Z is a hydroxy or amine group or an -OR1 group wherein R₁ is a C₁-C₄ alkyl or a C1-C4 acyl;
p is an integer and 13 > p ≥ 2; and
q is an integer and 13 > q ≥ 2, preferentially p+q <23;
on the condition that:
if p =4, then q ≠ 8;
if p =10, then q ≠ 4; and
if p =8, then q ≠ 6.

5. The compound of formula (A) according to claim 4 **characterized in that**:
- Z is -OH or OAc;
- Y is Cl;
- X is Cl; and
- p and q are even numbers with preferentially 8 ≥ p ≥ 2 and 10 ≥ q ≥ 2.

6. The compound of formula (A) according to claim 4 or 5 **characterized in that**:
- p =2 and q =2, 4 or 10, preferentially 4;
- p =4 and q =2, 4 or 6, preferentially 4;
- p =6 and q =2, 4, 6 or 10, preferentially 4; or
- p =8 and q =4 or 8, preferentially 4.

7. A process for producing the compound of formula (A) **characterized in that** the compound of formula B: 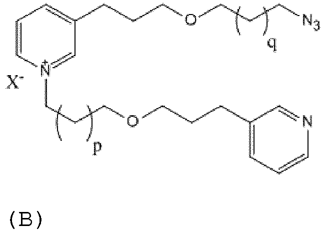 wherein X, p and q are as defined in any one of claims 4 to 6, is reacted in the presence of a catalyst, preferentially copper, with the compound of formula C: 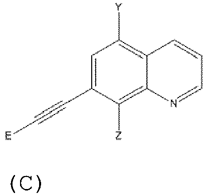 wherein Y and Z are as defined in any one of claims 4 to 6, and E is a leaving group selected from the group consisting of trimethylsilyl, tri-isopropyl silyl (TIPS) and dimethyl alcohol.

8. A compound of formula (D): 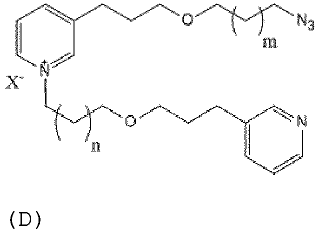 **characterized in that**:
X is a halogen;
m is an integer and 13 > m > 2; and
n is an integer and 13 > n ≥ 2, preferentially 23 > m+n, and the pharmaceutically acceptable salts.

9. The compound of formula (D) according to claim 8 **characterized in that**:
- X is Cl;
- m and n are even numbers; and/or
- preferentially m+n > 6.

10. The compound of formula (D) according to claim 8 or 9 **characterized in that**:
- m =4 and n =6 or 8, preferentially 6;
- m =6 and n =8 or 10, preferentially 10; or
- m =8 and n =4, 6, 8, or 10, preferentially 6 or 10,
- m =10 and n= 4 or 10, preferentially 10.

11. The compounds of formula (A) according to one of claims 4 to 6 and/or (D) according to one of claims 8 or 9 as a medicinal product.

12. The compounds of formula (A) according to one of claims 4 to 6 and/or (D) according to one of claims 8 or 9 for use in preventing and/or treating a pathogenic infection caused by *Pseudomonas aeruginosa.*

13. The compounds of formula (A) according to one of claims 4 to 6 and/or (D) according to one of claims 8 or 9 for use according to claim 12 **characterized in that** the pathogenic infection is a nosocomial infection, preferentially selected from eye infections, wound infections, in particular following a burn and/or an operation, urinary infections, in particular infections of the bladder or the urethra for example after catheterization, infections of the gastrointestinal system, lung infections in particular after bronchoscopy, meningitis in particular from inoculation, septicemia.
